# EUROPEAN PATENT SPECIFICATION

(11) **EP 2 542 689 B1**
(45) Date of publication and mention of the grant of the patent: **10.06.2015**
(21) Application number: 11705620.0
(22) Date of filing: 01.03.2011
(51) Int. Cl.: C12Q 1/68, G01N 33/49, G01N 33/569, G01N 33/574

(54) **Method for isolating target cells**
Verfahren zur Isolierung von Zielzellen
Nanocomposites dotés d'une homogénéité améliorée

(30) Priority: 02.03.2010 EP 10155178
(43) Date of publication of application: 09.01.2013
(73) Proprietor: XanTec bioanalytics GmbH, 40225 Düsseldorf (DE)
(72) Inventor: DANKBAR, Nico, 40589 Düsseldorf (DE); BRANDT, Burkhard, 25421 Pinneberg (DE); GEDIG, Erk, Tjalling, 40225 Düsseldorf (DE)
(74) Representative: UEXKÜLL & STOLBERG
(86) International application number: PCT/EP2011/053052
(87) International publication number: WO 2011/107489

(56) References cited:
- EP-A1- 0 630 675
- EP-A1- 1 484 390
- EP-A2- 1 018 346
- WO-A1-98/45413
- YAMADA TAKASHI ET AL: "Removal of malignant cells through a leukocyte-depletion filer for autologous blood transfusion", INTERNATIONAL JOURNAL OF CLINICAL ONCOLOGY, vol. 2, no. 3, September 1997 (1997-09), pages 143-146, XP002594056, ISSN: 1341-9625
- GALANZHA EKATERINA I ET AL: "In vivo magnetic enrichment and multiplex photoacoustic detection of circulating tumour cells.", NATURE NANOTECHNOLOGY DEC 2009 LNKD- PUBMED:19915570, vol. 4, no. 12, December 2009 (2009-12), pages 855-860, XP002594057, ISSN: 1748-3395
- SUEOKA A: "Present status of apheresis technologies: part 4. Leukocyte filter.", THERAPEUTIC APHERESIS : OFFICIAL JOURNAL OF THE INTERNATIONAL SOCIETY FOR APHERESIS AND THE JAPANESE SOCIETY FOR APHERESIS FEB 1998 LNKD- PUBMED:10227794, vol. 2, no. 1, February 1998 (1998-02), pages 78-86, XP002594058, ISSN: 1091-6660
- NÉRON SONIA ET AL: "Whole-blood leukoreduction filters are a source for cryopreserved cells for phenotypic and functional investigations on peripheral blood lymphocytes.", TRANSFUSION APR 2006 LNKD- PUBMED:16584429, vol. 46, no. 4, April 2006 (2006-04), pages 537-544, XP002594059, ISSN: 0041-1132
- NAGARSHETH NIMESH P ET AL: "Blood salvage use in gynecologic oncology.", TRANSFUSION OCT 2009 LNKD- PUBMED:19555419, vol. 49, no. 10, October 2009 (2009-10), pages 2048-2053, XP002594060, ISSN: 1537-2995
- WIESEL M ET AL: "[Separation of urologic tumors cells from Cell Saver blood using a membrane filter. A new method in autotransfusion?]", DER UROLOGE. AUSG. A MAY 1992 LNKD- PUBMED:1615592, vol. 31, no. 3, May 1992 (1992-05), pages 182-185, XP008124870, ISSN: 0340-2592
- BURKHARD BRANDT ET AL: 'Innovationswettbewerb Medizintechnik 2009 Modul "Transfer" "MetaCell" Modulares Nachweissystem für Tumorzellen in Körperflüssigkeiten', [Online] 05 November 2009, XP055089743 Retrieved from the Internet: <URL:http://www.innovationsforum-medizintec hnik.de/downloads/dokumentation_09/transfer _gewinnervortrag_brandt.pdf> [retrieved on 2013-11-22]
- ERK T GEDIG: 'Surface Chemistry in SPR Technology', [Online] 16 January 2008, XP055089747 Retrieved from the Internet: <URL:http://turroserver.chem.columbia.edu/s urfaceplasmons/Handbook/Chapter6_SPR.pdf> [retrieved on 2013-11-22]

### SEQUENCE LISTING

<110> Universitaetsklinikum Hamburg-Eppendorf
<120> Method for isolating target cells
<130> P 84891
<160> 6
<170> PatentIn version 3.3
<210> 1
   <211> 22
   <212> DNA
   <213> Artificial
<220>
   <223> oligonucleotide primer
<220>
   <221> misc_feature
   <222> (1). (1)
   <223> 6-Fam label at 5' end
<220>
   <221> misc_feature
   <222> (1)..(1)
   <223> 6-FAM label at 5' end
<400> 1
   gtttgaagaa tttgagccaa cc 22
<210> 2
   <211> 21
   <212> DNA
   <213> Artificial
<220>
   <223> oligonucleotide primer
<400> 2
   ttctgtctgc acacttggca c 21
<210> 3
   <211> 20
   <212> DNA
   <213> Artificial
<220>
   <223> oligonucleotide primer
<400> 3
   tctgcattcc tgccgagttc 20
<210> 4
   <211> 21
   <21-2> DNA
   <213> Artificial
<220>
   <223> oligonucleotide primer
<400> 4
   gcagtctcea ctccatgctc a 21
<210> 5
   <211> 20
   <212> DNA
   <213> Artificial
<220>
   <223> oligonucleotide primer
<400> 5
   aaagccgctc aactacatgg 20
<210> 6
   <211> 21
   <212> DNA
   <213> Artificial
<220>
   <223> oligonucleotide primer
<400> 6
   tgctttgaat gcgtcccaga g 21

## Description

The present invention relates to a method for the enrichment and/or isolation of target cells in a sample, which sample comprises red blood cells and/or platelets, said method comprising (a) filtering the sample through a filter element having a pore or mesh size of between 0.5 and 5 µm, (b) contacting the cells retained by the filter element in step (a) with a separation surface, wherein said separation surface is coated with a hydrogel and comprises affinity molecules which selectively bind to the target cells, (c) incubating the cells and the separation surface under conditions which allow for the binding of the affinity molecules to the target cells; and (d) separating the separation surface from any unbound cells and material.

### BACKGROUND OF THE INVENTION

Disseminated tumor cells (DTC), i.e. tumor cells which are detectable in the peripheral blood or in the bone marrow of a cancer patient, have been shown in clinical studies to provide informative value in terms of prognosis of a cancer patient as well as the evaluation of therapeutic outcome. Apart from this, these cells in many cases have the capability to indicate the occurrence of a tumor at an early phase of the disease.

For example, studies with breast cancer patients revealed that the detection of disseminated tumor cells in the blood of patients was significantly correlated with a reduced progression-free survival and overall survival (Gaforio et al. (2003) Int. J. Cancer, 107(6):984-90; Statho-poulou et al. (2002) J. Clin. Oncol., 20(16):3404-12). Similar results were obtained from studies performed in other types of cancer (Koch et al. (2005) Ann. Surg., 241(2):199-205; Kinele et al. (2003) Ann. Surg., 238(3):324-30). Accordingly, the accurate monitoring of disseminated tumor cells which are present in the blood or bone marrow of cancer patients provides useful parameters for therapy optimization and for predicting the course of the disease.

Moreover, the haematogenous spreading of tumor cells from a primary tumor is also assumed to play a role in the development of metastases (Eccles S.A. & Welch D.R. (2007), Lancet, 369, 1742-1757). Metastasis formation rather than progression of the primary tumor itself is the main cause of death in tumor diseases. Thus, the detection of early signs for metastasis formation is crucial for the treatment regimen to be applied to a cancer patient.

At present, the detection and isolation of disseminated tumor cells is routinely performed by use of magnetic beads which comprise antibodies that bind to defined surface antigens of the tumor cells. These modified beads are incubated with a blood sample of an individual to specifically bind tumor cells which are present in the sample. However, the current available techniques are associated with a high degree of unspecific cell adhesion to the beads as well as a low detection rate of tumor cells. It is known that due to the high number of non-target cells - in particular red blood cells - in the sample, it cannot be ensured in the routinely used methods that each tumor cell in a sample can interact with the antibody-presenting surface of a bead for a sufficiently long time so that binding between the immobilized antibody and the target cell occurs. In contrast, a significant number of tumor cells in a sample will not get in contact with their corresponding capture molecules.

For this reason, attempts were made in the prior art to remove red blood cells so as to enrich the tumor cells in the sample. For example, a density gradient centrifugation using specific density gradient media (such as Ficoll, Nycodens, Nycoprep, and the like) was used. The media employed for this purpose have densities which are between the density of the red blood cells and nucleated cells. Upon centrifugation, red blood cells and nucleated cells become enriched in different phases of the media and can be separated from each other by pipetting. This approach has the particular disadvantage that it is labor-intensive and requires highly experienced personnel for performing the separation. Moreover, disseminated tumor cells may exhibit a broad density range which means that part of these cells may not be available for a subsequent affinity binding step. The use of density gradient media also requires several washing steps of the tumor cells which are associated with several adverse effects, such as shear stress and unspecific adsorption of the cells to the wall of the reaction tubes, both of which may result in a loss of target cells. It has been demonstrated that the enrichment of tumor cells by density gradient centrifugation regularly results in a loss of almost two-third of the tumor cells present in the sample (Choesmel et al. (2004), 101, 693-703). Furthermore, density gradient centrifugation does not allow the processing of larger sample volumes, which is a clear disadvantage when considering that disseminated tumor cells are present in the blood or bone marrow in extremely low concentrations.

In an alternative approach that is currently used, red blood cells are lysed by the addition of lysis buffer, such as ammonium chloride, to the blood sample. By adding lysis buffer the membranes of the red blood cells are disrupted which results in cell death and release of intracellular components. However, it has been demonstrated that the use of lysis buffers regularly also disrupts a considerable portion of the disseminated tumor cells present in the sample. Furthermore, it can be assumed that the use of these buffers will adversely affect the metabolism of the target cells, which could be disadvantageous for further processing steps (e.g. culturing the isolated cells). The "Metacell" approach by Brandt and Gedig (2009) uses preselection steps and coated lydrogels.

In light of the above-mentioned problems, it is an object of the present invention to provide an improved method for enriching and/or isolating target cells which are present in a biological sample together with red blood cells and/or platelets, said method having a high recovery rate and at the same time involving a gentle treatment of target cells so that only few target cells are disrupted during the process. In particular, the process should not impose extensive mechanical or chemical stress on the target cells. It is also an object of the present invention to provide a method that allows for a highly specific binding of target cells in blood samples to separation surfaces, which method is characterized by a reduced unspecific adhesion of non-target cells, such as red blood cells, to said surfaces. These objects and other advantages are achieved by the methods defined in the enclosed claims.

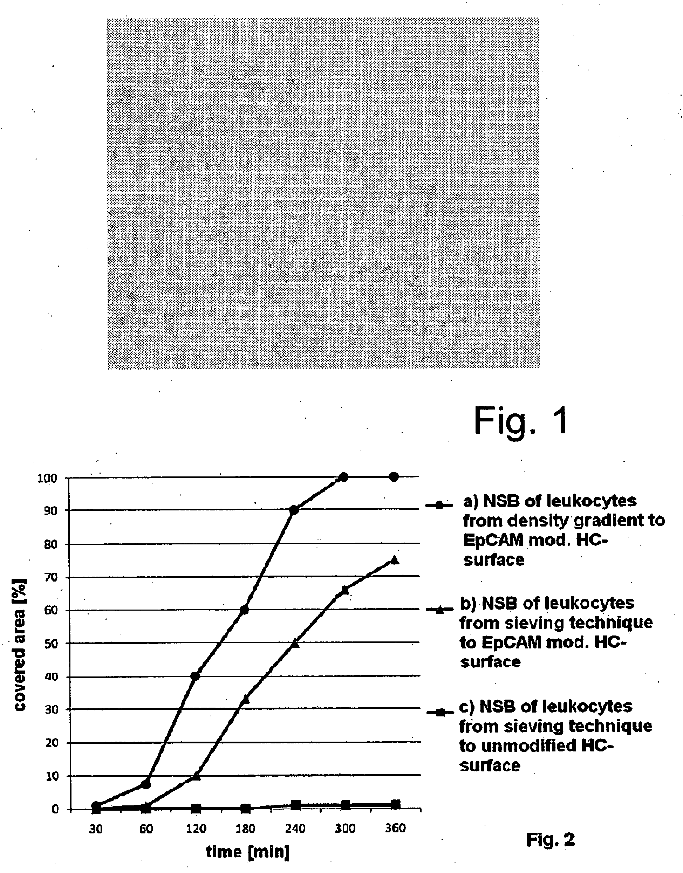

It has been found herein that a combined method comprising the filtration of a sample which contains red blood cells and/or platelets (such as a whole blood sample) through a filter element having a particularly small pore size and the subsequent separation of the target cells in the retentate via separation surfaces to which the target cells specifically bind, results in an extremely high recovery rate. In the examples of the present invention, almost 100% of the labelled target cells that were subjected to the method of the invention could be recovered. The unspecific adhesion of red blood cells, platelets or leukocytes to the separation surfaces, which could interfere with the affinity binding step, is considerably reduced by the initial filtration step. Also, it has been found that leukocytes which have been separated by the filtering step according to the invention, show a factor 2 lower non-specific binding affinity to the separation surface when compared to a leukocyte in a reference sample in which red blood cells were depleted by density gradient centrifugation (see Figure 2). Accordingly, the recovery of target cells by the separation surface (also referred to as panning) is much higher than that achievable by the methods in the prior art.

Thus, in a first aspect, the present invention discloses a method for the enrichment and/or isolation of target cells in a sample, which sample comprises red blood cells and/or platelets, comprising
(a) filtering the sample through a filter element having pores, holes or apertures with a size of between 0.5 and 5 µm;
(b) contacting the cells retained by the filter element in step (a) with a separation surface, wherein said separation surface is a hydrogel coating on a support material and comprises affinity molecules which selectively bind to the target cell;
(c) incubating the cells and the separation surface under conditions which allow for the binding of the affinity molecules to the target cells; and
(d) separating the separation surface from any unbound cells and material.

The method of the invention can be applied to any sample that comprises red blood cells and/or platelets. Preferably, the sample comprises a body fluid or a tissue extract from an individual. Preferably, the individual from which the sample is derived is a vertebrate, and more preferably, a mammal. In a particularly preferred embodiment, the sample is derived from a human. The sample will normally be a cell suspension. The sample can comprise or consist of blood (e.g. whole blood) or blood components, urine, pleural effusions, ascites, bronchoalveolar lavage, nipple aspirate of the gland of the female breast or bone marrow. In a preferred aspect, the sample comprising the target cells is a blood or bone marrow sample, e.g. a sample from a human individual comprising or mainly consisting of blood or bone marrow, respectively. The sample can be taken by any suitable method known in the prior art. For example, if the sample is a human blood sample, it can be taken by vein puncture. Methods for obtaining bone marrow, e.g. human bone marrow, are also well known in the art. For example, red bone marrow in admixture with blood can be harvested from the crest of the ilium or from the sternum, an intervention which is generally performed by minimal invasive surgery. Alternatively, the biological sample can be ascites, i.e. peritoneal cavity fluid, pleural effusion, aspirates from the female breast nipples, urine and other body fluids.

When the sample to be used in the method according to the invention has a particularly high viscosity, it may be desirable to dilute it prior to or simultaneously with applying the sample to the filter element. For this purpose, any physiologically acceptable buffer may be used which does not interfere with the subsequent binding of the target cells to the affinity separation surface. Suitable buffers for the dilution of the samples are iso-osmolaric buffers which buffer within physiological pH, for example, phosphate buffered saline (PBS), Hank's balanced salt solution, Tris-buffered saline, HEPES-buffered saline, MES buffer and the like. The pH of the physiological buffer is preferably within the range of from about pH 6.0 to about 9.0, more preferably between about pH 6.5 to about 8.0, and most preferably between about pH 7.0 to about 7.5, for example 7.4.

One particular advantage of the method of the present invention is that considerably high volumes of a sample can be processed. For example, a sample volume of at least 5 ml or more, e.g. 8 ml, 10 ml, 15 ml, 20 ml, 25 ml, 30 ml, 35 ml, 40 ml, 45 ml, 50 ml, 60 ml, 70 ml, 80 ml, 90 ml or even 100 ml, 150 ml, 200 ml, 500 ml, 1000 ml or more can be subjected to the filtration step before the retentate is then contacted with the separation surface. In this way, the filtration step can be used to concentrate the retentate, leading to reduced sample processing time and increased overall performance of the method. In contrast, currently available methods rely on the use of magnetic beads which are added to a sample that is suspected to comprise target cells. In these methods, only small volumes of between 2-5 ml sample fluid can be incubated with the magnetic beads, because otherwise the likelihood of a contact between the target cells and the antibodies on the surface of the particles significantly decreases, thereby leading to low recovery rates. The initial filtration step enriches the target cells and, at the same time, removes a considerable number of red blood cells and/or platelets, which would otherwise interfere with the subsequent binding of the target cells to the affinity molecules on the separation surface.

In a first step of the method of the invention, the sample is filtered through a filter element having pores, holes or apertures with a size of between 0.5 and 5 µm. By use of the filter element red blood cells and/or platelets are removed from the sample which could otherwise interfere with binding of the target cells, e.g. disseminated tumor cells, to their corresponding affinity molecules on the separation surface. Red blood cells (erythrocytes) are non-nucleated blood cells having a mean diameter of 5-8 µm which are responsible for the oxygen transport in the blood. It has been found that due to the high deformability of these cells, filter elements with a pore size of as little as 0.5 µm can effectively be used for the removal of the vast majority of these cells. Red blood cells have a flattened shape and can form tubular structures that are able to pass through apertures having a size which is significantly below the average diameter of the red blood cells in their relaxed, flattened state.

Similarly, platelets are cells without a nucleus that usually have a mean diameter of between 1-2 µm and some degree of deformability which means that they are also capable of passing through the pores of the filter elements having the above-mentioned pore size. In contrast, nucleated cells such as leukocytes or tumor cells are generally larger than 5 µm and have a limited ability to deform. Those cells will be retained by the filter element contemplated for use in the method of the invention. As shown in the below example 6, it was readily possible by selection of the appropriate filter element to separate tumor cells and leukocytes from the majority of red blood cells and platelets in a human whole blood sample, without any considerable loss of tumor cells.

The filtration in step (a) of the above method removes a considerable portion of the red blood cells present in the sample. Preferably, the filtration process removes about 30%, about 40%, about 50%, about 60%, about 70%, about 80%, about 90%, about 95% or even up to about 96%, about 97%, about 98%, about 99% or more of the erythrocytes which are present in the sample. The filtration process in step (a) is likewise suitable to remove a considerable portion of the platelets present in the sample. Preferably, the filtration process removes about 30%, about 40%, about 50%, about 60%, about 70%, about 80%, about 90%, about 95% or even up to about 96%, about 97%, about 98%, about 99% or more of the platelets present in the sample. Nucleated cells such as leukocytes or tumor cells are retained by the filter element, so that the filtration can be used to enrich the fraction of these cells relative to red blood cells or platelets, respectively.

As used herein, the term "filter element" refers to a medium or material comprising regularly or irregularly distributed pores that allow for the passage through the filter element of cells or compounds below a certain threshold size or diameter, while retaining other cells, aggregates or compounds having a size or diameter exceeding the threshold size or diameter. The filter element for use in the practise of the present method can include all kinds of commonly used filter media or materials and can have any shape and size. For example, the filter element, which is preferably sheet-shaped, may consist of or comprise one or more woven or non-woven fabrics, one or more perforated sheets, one or more screens or meshes, one or more microporous materials, one or more membranes or a combination of two or more of such materials. As commonly used in filter technology, the term "pore" denotes holes, openings and apertures provided in all of the above materials. Thus, for example, in the case of a wire mesh or woven fabric the pores are the individual meshes or mesh apertures with the pore size being the mesh size, and in the case of a screen the pores are the individual screen apertures. It is preferred that the filter element comprises or consists of a single layer of one of these materials or comprises or consists of at least two layers of these materials. For example, the filter element may comprise or consist of one or more membranes or membrane filters, such as those which are commonly used for filtering particulate matter from a liquid or gaseous fluid.

In any case, according to the invention, the pore size of the filter element is between 0.5 and 5 µm, e.g. 0.5, 1, 2, 3, 4 or 5 µm. Preferably, the pore size of the filter element is between 1 and 2 µm. In this regard, the size of a particular pore is understood herein as the minimum diameter across that pore. In other words, the pore size as used herein is identical to the diameter of the largest spherical body that is still able to pass through the pore.

In particular, the filter element may comprise or consist of a woven or non-woven fabric. As used herein the term "non-woven fabric" refers to a web-like structure, wherein randomly orientated fibers, filaments or threads are interlaid in a non-aligned or random manner. The non-woven filters may be comprised of any material that does not substantially interfere with the object of separating red blood cells and platelets from the target cells. In particular, the material should not be such that any substantial adhesion of the red blood cells or platelets occurs which would prevent an effective passage of those cells through the filter element. Similarly, the material should also not allow for any substantial unspecific adhesion of target cells, for example, disseminated tumor cells, to the filter material, which would impede the subsequent transfer of the target cells to the separation surface. Filter elements made of non-woven fabrics which allow for cell separation are well known in the art, and they may consist of various materials, such as cellulose or cellulose derivatives, including, cellulose acetate, cellulose nitrate, hydroxypropyl cellulose (HPC), hydroxyethyl cellulose (HEC), hydroxybutylmethyl cellulose, hydroxypropylmethyl cellulose. Other commonly used materials for non-woven filters include, polycarbonate, hydroxyethyl starch, polysulphones, polyethersulphones, polyamides, polyether ketones, polyetherimides, polyarylenes, polyphenylene ethers, polyvinylidene fluoride (PVDF), polytetrafluorethylene (PTFE) and the like as well as glass. Non-woven filter materials can be made by various known processes, for example, meltblowing, spunbonding, air-laying, wet-forming and bonded carded web methods. The production of non-woven fabrics is described, for example, in US Patent Nos. 4,340,563 and 3,849,241. Suitable non-woven filters are also available from several different suppliers, for example, from Carl Roth (Karlsruhe, Germany), Schleicher & Schuell (Dassel, Germany) and Satorius (Göttingen, Germany).

In a particular preferred embodiment, the filter element used in the methods of the invention comprises or consists of a woven fabric. As used herein, a "woven fabric" refers to a material which has been prepared by a weaving process, i.e. by interlacing of warp and weft fibers, filaments or threads. The warp and weft fibers, filaments or threads cross, preferably at essentially right angles, in turns above or below each other. This preferably results in a textile with square or rectangular mesh openings having a defined mesh size which can be used for separating particles with a defined size. According to the invention, the pore or mesh size of the woven filter element is between 0.5 and 5 µm, e.g. 1, 2, 3, 4 or 5 µm, wherein the size of a particular mesh or aperture is understood herein as the minimum diameter across that mesh or aperture. In other words, the mesh or aperture size as used herein is identical to the diameter of the largest spherical body that is still able to pass through the mesh or aperture. Woven fabrics suitable for use in the present methods include screening fabrics, as those commonly used in silk screen or offset printing methods. The woven fabric for use in the present method may in particular comprise or consist of polyamide, aromatic polyamides, polyethylene, polypropylene, polyester, polyfluorocarbon, polyacrylonitrile, polyurethane, polyacrylate, nylon, polyphenylene sulfide, polytetrafluoroethylene, polybenzimidazole, and the like. Suitable woven filters are purchasable from different suppliers, for example, the monofilament textile polyamid 6.6 monofil from Schwegmann Filtrations-Technik GmbH, Gelsdorf, Germany; PA1/1-PA 5/1 from Franz-Eckert GmbH, Waldkirch, Germany; Sefar Nitex 03-1/1 or Sefar Nitex 03-5/1 or Sefar Petex 07-5/1 from Sefar AG, Heiden, Switzerland.

The woven or non-woven filter elements may additionally be coated with bioinert layers which suppress any interaction with the cells present in the sample. These bioinert layers can comprise, for example, suitable blocker proteins such as BSA or casein or a hydrophilic coating such as a hydrogel, or a hydrophilic oxidized layer which may be prepared by (plasma) oxidation of organic filter elements.

Further preferred filter elements which can be used in the methods of the present invention include micro sieves made by etching silicon wafer-based materials, such as silicon nitride. The sieves are prepared by advanced semiconductor methods which provide a very thin separation layer and uniformly sized pores. By use of this technology pores and apertures having a diameter of 0.35 µm can be prepared. Suitable filter elements can be purchased, for example, by fluXXion B.V., (Eindhoven, The Netherlands) or by Aquamarijn Micro Filtration B.V. (Zutphen, The Netherlands) under the tradename microsieve^{®}.

Other suitable filter elements include polymer structures prepared by a phase separation moulding process. This technology uses a mixture of a polymer in a solvent/non-solvent system to form a three-dimensional structure over a mould. By selecting the right blend of polymer and solvent/non-solvent, micro porous structure with pore sizes in the range of 0.5 µm to 1 µm can be produced. Moulded filter elements which are based on this technology include those provided by Aquamarijn Micro Filtration B.V. (Zutphen, The Netherlands).

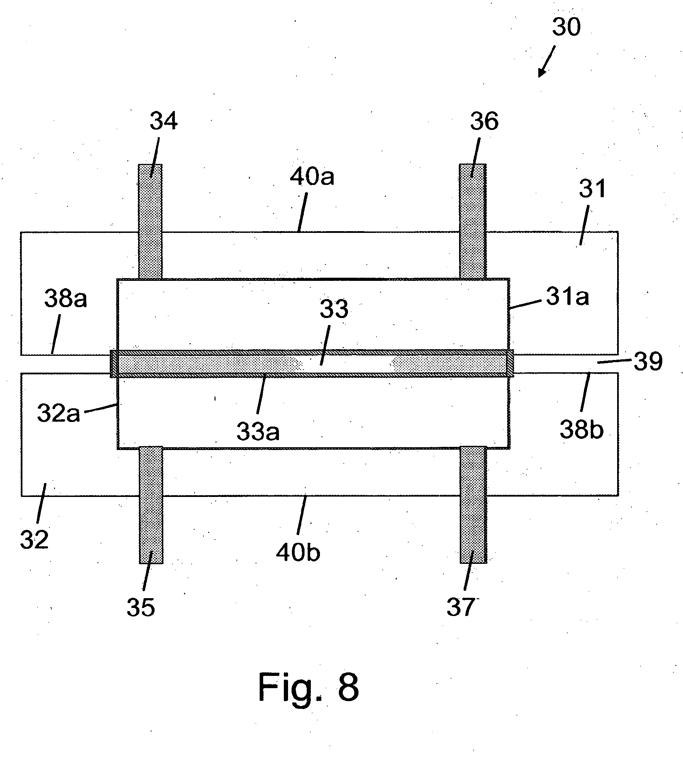

The sample or the diluted sample can be filtered through the filter element simply by providing a pressure difference between the two sides of the filter element, with the pressure being higher at the side to which the sample is provided and at which the retentate is formed. For example, such pressure difference may advantageously be created by suitably utilising gravity or by suitably utilising differences in hydrostatic pressure. Depending on the specific set-up selected for the filtration step, and depending on parameters of the filter element (in particular the pore or mesh size of the filter element) the skilled person will be able to adjust the pressure to be applied for conducting the sample through the filter element. Briefly, the pressure should be sufficiently high to effectively remove red blood cells and/or platelets which are present in the sample by forcing them to traverse the filter element. On the other hand, the pressure should be low enough so that the target cells are not damaged or pushed through the filter element. The filter element can be used in various forms, provided that the selected filter element can be fitted into the specific filtration process. For example, if a two-chamber assembly as defined below and exemplified in figures 8-9 is used, a filter element comprising one or more flat sheets is preferably used between the two chambers. Other forms of the filter element may be used as well, for example, funnel-shaped elements or v-shaped troughs.

Alternatively, the filtering step of the method of the invention can also be achieved merely on the basis of diffusion. For example, a set-up can be chosen where the sample comprising the platelets and/or red blood cells is separated by the filter element from a buffer solution which is devoid of platelets and/or red blood cells. The buffer can be, for example, any of the buffers mentioned above in the context of sample dilution. If incubated for a sufficient time period, the platelets and/or red blood cells present in the sample will pass the filter element to create equilibrium between the different concentrations in the sample and the buffer. This directed diffusion process can be maintained and supported by continuously removing any platelets and/or red blood cells which penetrate the filter element from said buffer (e.g. by replacing the buffer). In embodiments where the filtering is achieved by pure diffusion, it is also possible to use filter elements in the form of sealed bags which include the sample with the target cells. The sealed filter elements can be submerged into a suitable buffer medium (e.g. PBS) which is devoid of red blood cells and platelets. The red blood cells and/or platelets in the bag-like filter element will gradually penetrate into the surrounding medium. Preferably, the cells entering the medium are removed from, for example, by repeatedly or continuously substituting the medium containing the blood cells with fresh medium. This can be achieved, for example, by use of a peristaltic pump at a flow rate of 5 ml/ min.

For pre-filtration, a filter element having a pore size of 10-30 µm can be used in the filtering process according to the invention, in particular if the biological sample is a blood sample. The filter element for pre-filtration is used to remove cell aggregates which may form by aggregation of leukocytes and platelets or by interaction of fibrin, fibrinogen and other blood components. Thus, the methods of the present invention can include a step which precedes step (a) of the above method, in which the sample is filtered through a pre-filter element having a pore or mesh size of between 10 and 30 µm. The pre-filter element may comprise or consist of the materials mentioned above in connection with the filter element.

The target cells which the present method seeks to enrich and/or isolate are preferably cells that occur in low concentrations in the sample. According to a particular preferred embodiment, the target cells are tumor cells, preferably disseminated tumor cells, and more preferably human disseminated tumor cells. Disseminated tumor cells are cancerous cells which have detached from the primary tumor and circulate in the peripheral blood or become enriched in the bone marrow. These cells are present in the blood or bone marrow in very low concentrations, so that their detection and isolation is generally complicated. For example, tumor cells circulating in the blood are normally present in a concentration of about 1 in 10⁻⁶ to 10⁻⁸ leucocytes. In contrast, the concentration of red blood cells is in the range of 4-6 x 10⁹ cells per ml human blood. Platelets are present in the blood in a concentration of about 1,5-3,0 x 10⁶ cells per ml blood. The high number of red blood cells and platelets in the blood has the adverse effect that the binding of target cells, such as tumor cells floating in the blood stream, via specific binding molecules (e.g. antibodies) on a separation surface, for example, a magnetic particle or a coated support surface, is often inefficient, because most of the target cells do not sufficiently interact with their corresponding binding molecules to provide for a binding to the separation matrix.

The disseminated tumor cells to be detected by the method of the present invention can originate from a primary tumor of any cancer type, for example, from the following primary tumors: prostate cancer, cervical cancer, pancreatic cancer, breast cancer, colon cancer, brain cancer, lung cancer, bronchial cancer, liver cancer, bladder cancer, skin cancer, head and neck cancer, hematological cancers, cervical cancer, ovarian cancer, stomach cancer, kidney cancer, uterine cancer, bone cancer, esophageal cancer, laryngeal cancer, naso-pharyngeal cancer, oropharyngeal cancer, testicular cancer, vulvar cancer, hepatoma, salivary gland carcinoma, thyroid cancer, parathyroid cancer, lymphomas, sarcomas, gallbladder cancer, germ cell cancer, multiple myeloma, small intestine cancer, thymus cancer, and the like. Preferably, the cancer type from which the disseminated tumor cells are derived is a carcinoma, i.e. a tumor of epithelial origin, more preferably a breast, colon, lung or prostate carcinoma.

Apart from tumor cells, other cell types which are rarely present in the blood or bone marrow can be target cells, such as plasma cells, rare subtypes of lymphocytes, e.g. memory cells, blastocystic cells, fetal cells, placenta cells, and the like.

After separating the target cells from red blood cells and/or platelets, the cells which have been retained by the filter element are transferred to or brought into contact with a separation surface. Any method which is suitable to establish a contact between the cells in the retentate and the separation surface may be used. For example, where the separation surface is a coated slide, the target cells can be resuspended in a small volume of buffer (if necessary) and transferred by pipetting them onto the slide surface. The buffer can be, for example, any of the buffers mentioned above in the context of sample dilution. The aliquot containing the retentate will normally have a volume of between 0.5 and 8 ml, e.g. about 1, 2, 3, 4, 5, 6, or 7 ml. If necessary, the aliquot may be diluted with the above discussed buffers to give volumes of 10, 20, 20, 30, 40 or 50 ml. Alternatively, where the affinity separation surface is a small particle, such as a magnetic bead with antibodies immobilized thereon, the target cells can be resuspended in buffer and the beads are subsequently added to the cell suspension for further incubation. Also, the separation surface may be in the form of particles, for example glass particles or particles of a resin, so that the target cells which have been resuspended in a small volume of buffer can be conducted through a chromatography column filled with said particles. Also, the separation surface can have the form of a capillary through which a suspension comprising the target cells is conducted.

As used herein, the term "separation surface" broadly refers to any surface that carries at least one type of affinity molecule suitable to selectively bind a predetermined structure on the target cell. The separation surface can be a particle surface, for example, the surface of a bead, such as a magnetic bead or glass bead, which is modified by the immobilization of affinity molecules, such as antibodies, which are directed to defined antigenic structures of the target cells. Alternatively, the separation surface can be the surface of a substantially flat support, such as the surface of a slide, e.g. a glass or plastic slide. In a preferred example, the separation surface is part of a biochip, preferably made of glass or transparent plastic. The separation surface may also be present as a chromatography resin material. The above-mentioned support materials can be provided with a coating to render the separation surface bioinert. This means that cells are prevented from being adsorbed non-specifically to the surface. According to a particularly preferred aspect, the separation surface is coated with a natural or synthetic hydrophilic polymer layer. Preferably, the hydrophilic polymers form a three-dimensional structure on the support material which is highly hydrated. Accordingly, such polymer network is often referred to as "hydrogel". A hydrogel can contain up to 99% or more water, whereas the polymer content can be 1% or even lower. Methods for preparing hydrogels for use in the present invention are described, for example, in WO 02/10759. This international application, describes the use of adhesion mediator layers which provide for the coupling of the hydrogel polymers to the support material. The hydrogel coatings provided in WO 02/10759 are particularly preferred for use in the method of the present invention.

Various hydrophilic polymers can be used for the hydrogel coating. For example, the coating may comprise or consist of polysaccharides, polyalcohols, polyethers, pdyamides, polycarboxylic acids, polysulfates, polysulfonates, polyphosphates, polyphosphonates and/or combinations or functionalized derivatives thereof. Such functionalizations includes, for example, isothiocyanates, isocyanates, carboxylic acid azides, N-hydroxysuccinimides, N-acylimidazoles, sulfonylchloride derivatives, aldehyde, keto, glyoxal, oxirane, carbonate, arylhalogenide, imidoesters, anhydrides, halogenalkyls, halogenacyls, maleimides, aziridines, acryloyls, sulfhydryls, disulfides, diazoalkanes, diazoacetyls, imidazolylcarbamates, hydrazides, diazo, arylazides, benzophenones, diazopyruvates or diazirines. A further preferred functionalization involves nitrilotriacetic acid (NTA) derivates, so that ligands or antibodies can be immobilized by means of a metal chelate. Steptavidin and/or biotin derivatives are also suitable for functionalization. According to a preferred example, the hydrogel coating comprises or consists of polycarboxylate polymers. According to a further preferred example, the hydrogel coating has a slightly negative charge, as measured, for example, by Zeta potential determination.

The hydrogel coating can be of any thickness which allows for the capture of the target cells on the surface of the hydrogel. Preferably, the hydrogel coating has a thickness of between about 100 nm and about 5000 nm, preferably between about 500 nm and about 3000 nm, for example, about 600 nm, about 700 nm, about 800 nm, about 900 nm, about 1000 nm, about 1100 nm, about 1200 nm, about 1300 nm, about 1400 nm, about 1500 nm, about 1600 nm, about 1700 nm, about 1800 nm, or about 1900 nm. The thickness of the coating can be determined by routine methods available in the prior art, for example, by atomic force microscopy or ellipsometry.

The coating preferably provides a three-dimensional surface structure in which the chains of the hydrophilic polymer are aligned at least partly vertical to the substrate surface, i.e. brush-like. Due to their increased surface compared to planar structures, such brush-like hydrogel surfaces show a particularly enhanced immobilization capacity for biomolecules, such as antibodies and other affinity molecules which are capable of binding the target cells. It has been found that brush-like structured hydrogel coatings, in particular those which comprise or consist of certain polycarboxylate polymers, provide an excellent surface for selectively attaching cells to a solid support for subsequent detection and/or quantification. Preferred hydrogel surfaces for use in the present invention are available, for example, as HC or HCX coated slides from XanTec bioanalytics GmbH, Dusseldorf, Germany.

The separation surface comprises one or more different types of affinity molecules which selectively bind to the target cells in the sample, thereby allowing the separation of the target cells from any other cell present in the retentate after removal of the red blood cells and/or platelets. The "affinity molecules" are broadly understood as molecules which bind to pre-determined structures of the cells, preferably to protein or saccharide structures which are present on the surface of the target cells. The affinity molecules are preferably protein molecules, such as receptors, lectins, ligands, antibodies, antibody fragments, nucleic acids (such as aptamers), saccharide structures, or combinations thereof.

According to the present invention, the use of antibody molecules or antigen-binding fragments thereof is particularly preferred for target cell binding. In the context of the present invention, the term "antibody" includes monoclonal antibodies, polyclonal antibodies, multi-specific antibodies (for example, bispecific antibodies), anti-idiotypic antibodies, chimeric antibodies, and humanized antibodies, as long as they exhibit the desired immunological activity, i.e. specific binding to the target cells. In particular, the term is to be understood as comprising any kind of artificial antibody molecule that was prepared by grafting techniques and the like. The antibodies for use in the present method may be of any isotype class, such as IgG, IgM, IgA, IgE, and IgD as well as their subclasses, e.g., IgG1, IgG2 and the like.

Apart from whole antibodies, the present invention also refers to antigen-binding fragments of an antibody which specifically bind to the target cell. As used herein, an antigen-binding fragment of an antibody is a fragment which retains at least a part of the specific binding activity of the whole antibody molecule for the particular antigenic structure of the target cell, e.g. a disseminated tumor cell. Antigen-binding fragments of the invention may comprise Fv, Fab, F(ab') and F(ab')₂ fragments. Further included by the term "antigen-binding fragment" are single chain Fv antibody fragments and disulfide-linked Fv fragments (dsFv). Methods for the recombinant production of antibody fragments have been described in the prior art and comprise, for example, techniques such as phage display or ribosome display. The recombinant antibody fragments produced by these methods may be purified and tested for binding affinity and specificity to the target cells, for example, tumor cells.

The immobilization of specific affinity molecules to a hydrogel-coated separation surface can be done in different ways. The standard method, which is suitable for most applications, involves covalent coupling of the affinity molecules via amino or hydroxyl functionalities to a hydrogel preactivated with N-hydroxysuccinimide (NHS). As the immobilized affinity molecules are covalently bound to the hydrogel coating rather than adsorbed, the immobilization process is equally well suited for all kinds of affinity molecules, for example, antibodies, proteins, peptides, low MW compounds, nucleic acids, and the like, as long as these affinity molecules bear suitable reactive groups, such as amino, (di)sulphide or aldehyde moieties. For alternative coupling strategies, streptavidin, protein A, disulphide or hydrazide functionalized coatings are likewise available in the prior art. The buffers containing the affinity molecule can be either applied to the entire separation surface or spotted onto defined regions of said surface. It is readily possible to combine different affinity molecules on one substrate. In a preferred example, the hydrogel-coated separation surface comprises 2 or more affinity molecules having different binding specificities, such as 2, 3, 4, or 5 different antibodies.

The separation surfaces can of course also comprise different types of affinity molecules, such as different antibodies or antibody fragments which are directed against the same or different antigenic structures of the same target cell. Alternatively, where the simultaneous enrichment of different types of target cells is desired within the same process, the separation surfaces can also comprise different antibodies or antibody fragments which are directed against different target cells. Preferably, the overall density of affinity molecules on the separation surfaces is in the range of about 0.1 to about 100 ng/mm², for example, about 0.5 ng/ mm², about 1 ng/ mm², about 5 ng/ mm², about 8 ng/ mm², about 10 ng/ mm², about 15 ng/ mm², about 20 ng/mm², about 30 ng/mm², about 40 ng/mm², about 50 ng/mm², about 60 ng/mm², about 70 ng/mm², about 80 ng/mm², about 90 ng/mm², about 100 ng/mm² or even higher densities. It is particularly preferred that the above densities refer to affinity molecules which are antibodies or antibody fragments. It is also preferred that the binding capacities of the affinity molecules which are immobilized on the separation surface are not substantially affected by the coupling process. After immobilization to the separation surface, the affinity molecules, e.g. the antibodies or antibody fragments, retain more than 50%, preferably more than 60%, more than 70%, more than 80%, more than 90%, and more preferably more than 95% of their original binding capacity for the target molecule, i.e. the binding capacity for the target without immobilization.

The affinity molecules, e.g. the antibodies or the antigen-binding fragments derived therefrom, are selected based on their capacity to exhibit selective binding to the target cells, e.g. to tumor cells. As used herein, selective binding means that the affinity molecule binds to the target cell at least about 4-fold stronger, usually more than about 5-fold stronger, more than about 6-fold, more than about 8-fold, more than about 10-fold, more than about 15-fold, more than about 20-fold, more than about 50-fold, or even more than about 100-fold stronger relative to the binding to a non-target cell in the sample, as reflected, for example, by the K_{D} value of the affinity molecule/target ligand pair. For example, when using a whole blood sample in the method of the invention, the affinity molecule should not exhibit any substantial binding to leukocytes which are present in the filtrate obtained in step (a) of the method.

In a further aspect, the affinity molecules on the separation surfaces can be receptors, lectins, ligands or functional portions thereof. In addition, affinity molecules with a low affinity to structures on the target cells can be employed. Although one interaction alone will generally not be stable enough for a separation process, the cooperative effect of several weak interactions distributed over the contact area of one cell with the separation surface results in a sufficiently strong and specific interaction. This is a significant advantage over state of the art nanoparticle based separation techniques, as the relatively small surface area of those particles is too small to allow for such a cooperative mechanism.

In a preferred example, the target cells to be enriched and/or isolated are disseminated tumor cells which are bound to a separation surface, for example, a hydrogel-coated support surface, via antibodies or fragments thereof which have a specific binding affinity for cell surface marker of these tumor cells. Suitable surface marker of disseminated tumor cells are well known in the art and include the epidermal growth factor receptor (EGFR), the epithelial cell adhesion molecule (EpCAM or CD326), the insulin growth factor-1 receptor (IGF-1 R), the epidermal growth factor receptor 2 (Her2), cytokeratin 19 (CK19), cytokeratin 20 (CK20), mucin 1 (MUC1), mucin 2 (MUC2), human Epithelial Membrane Antigen (EMA), epithelial antigen (Ber-EP4), folate receptor alpha (FRalpha). Further markers are discussed extensively in the literature, see, for example, Pantel et al. (2008), Nature Reviews, 8, 1-12; Pantel et al. (2009), Nat Rev Clin Oncol., 6(6):339-51.

Accordingly, antibodies for use as affinity molecules in the method comprise an anti-EpCAM antibody, such as antibody clone 323A3 (available from Kamiya Biomedical Company, Seattle, USA), antibody clone MK-1-25 (available from Acris, Herford, Germany), antibody clone AUA1 (available from Novus Biologicals, Littleton, USA), antibody clone 158206 (available from R'n'D Systems GmbH, Wiesbaden, Germany), antibody clone 528 (available from Santa Cruz Biotechnology Inc., Heidelberg, Germany), an anti-IGF-1R anti-body, such as CP-751,871 (available from Pfizer Pharma AG, Berlin, Germany), an anti-CD19 antibody, such as those available from Santa Cruz Biotechnology Inc., Heidelberg, Germany referred to as catalogue numbers sc-70563, sc-18895, sc-70560, sc-70559, sc-70561, sc-21714, sc-65295, sc-52311, sc-69736, sc-65255, sc-8498, sc-52378, sc-20922, sc-18884, sc-18894, sc-19650, sc-51529, sc-8500-R, sc-13507, sc-53191, sc-8499, sc-18896 and sc-69735); an anti-MUC1 antibody, such as those available from Santa Cruz Biotechnology Inc., Heidelberg, Germany referred to as catalogue numbers sc-71611, sc-71610, sc-71612, sc-71613, sc-59931, sc-71614, sc-59794, sc-59795, sc-59796, sc-59797, sc-52347, sc-6827, sc-53376, sc-59798, sc-52085, sc-59799, sc-53377, sc-6826, sc-59793, sc-59800, sc-15333, sc-25274, sc-53379, sc-52086, sc-52087, sc-52088, sc-52089, sc-52090, sc-52091, sc-52092, sc-52093, sc-6825, sc-53380, sc-73595, sc-53381, sc-56441, sc-65589, sc-65220, sc-69644, sc-73606, sc-80889, sc-73605, sc-7313, and sc-52094; an anti-MUC2 antibody, such as those available from Santa Cruz Biotechnology Inc., Heidelberg, Germany referred to as catalogue numbers sc-59859, sc-7314, sc-15334, sc-23170, sc-23171, and sc-13312; an anti-CK19 antibody, such as those available from Santa Cruz Biotechnology Inc., Heidelberg, Germany referred to as catalogue numbers sc-53258, sc-53257, sc-33110, sc-33120, sc-25724, sc-33111, sc-33119, sc-53003, and sc-56371; an anti-CK20 antibody, such as those available from Santa Cruz Biotechnology Inc., Heidelberg, Germany referred to as catalogue numbers sc-25725, sc-17112, sc-52320, sc-70918, sc-56522, sc-56372 and sc-58730; an anti-epithelial membrane antigen antibody, such as clone E29 of Dako Deutschland GmbH, Hamburg, Germany; an anti-epithelial antigen antibody, such as clone Ber-EP4 of Dako Deutschland GmbH, Hamburg, Germany; an anti-egfr antibody, such as sc-120 available from Santa Cruz Biotechnology Inc., Heidelberg, Germany. Further suitable antibodies are, for example, antibody VU-1 D9 from Novocastra Deutschland, Berlin, Germany; antibody Ks5+8.22/C22 from Progen Biotechnik GmbH, Heidelberg Germany; antibody A45-BB3-Cy3 from Micromet, Munich, Germany; antibody Mov18/Zel from Enzo Life Sciences GmbH, Lörrach, Germany. Also contemplated is the use of antigen-binding fragments of the above antibodies.

In one example of the disclosed method, the target cells in the sample can be preincubated with a suitable affinity molecule, e.g. the above mentioned antibodies or antibody fragments, prior to contacting the target cells with the separation surface. For example, the target cells can be pre-incubated in an initial step with one or more antibodies or antibody fragments which are directed to certain surface markers of the target cell. The separation surface comprises immobilized affinity molecules which are in turn directed to the affinity molecules used for the pre-incubation. For example, where the affinity molecules used for the pre-incubation of the target cells in the sample are mouse antibodies, then a suitable separation surface would comprise anti-mouse antibodies or fragments thereof which capture the mouse antibodies which have been attached to the target cells in the pre-incubation step. The same principle can be utilized by pre-incubating the sample containing the target cells with biotinylated antibodies, such as biotinylated IgG antibodies, which are directed to specific surface antigens of the respective target cells. These labelled antibodies can be captured by use of separation surfaces which are modified to comprise affinity molecules which selectively bind to the biotin residue of the antibodies, such as streptavidin. Preferably, any unbound biotinylated antibody is removed from the sample after the pre-incubation step, e.g. by the filtration step of the method.

After contacting the cells obtained from the retentate with the separation surface, the cells are incubated with the surface under conditions suitable to allow for the binding of the cells to their corresponding affinity molecules. Depending on the nature of the separation surface and the affinity molecules selected for capturing the target cells, the incubation conditions can vary. The skilled person will readily be able to identify the optimum parameters to be applied for a given separation surface. For example, the incubation times can vary from about 5 min to about 10 hours, but will normally in the range of between about 30 min and about 8 hours, preferably between about 2 and about 6 hours, e.g. 3, 4, or 5 hours. The temperature will be in the range of about 10°C to about 40°C, preferably from about 20°C to about 40°C, more preferably from about 32°C to about 36°C. In a particular preferred aspect, the incubation of the sample containing the target cells will be performed at ambient temperature, e.g. at 22°C to 25°C.

In a simple example, a liquid aliquot containing the retentate from the filtering step is transferred onto the separation surface, e.g. by pipetting, and subsequently incubated. The aliquot containing the retentate will normally have a volume of between 0.5 and 8 ml, e.g. about 1, 2, 3, 4, 5, 6, or 7 ml. If necessary, the aliquot may be diluted with the above discussed buffers to give volumes of 10, 20, 20, 30, 40 or 50 ml. The incubation does not necessarily have to involve agitation of the separation surface. However, agitation of the separation surface is preferred for an improved binding of the target cells to the surface. For example, where the separation surface is a substantially flat slide, the incubation can be performed by placing the slide in a Petri dish or a cytospin tube which is agitated on a rocking platform shaker with rotation speed between 0.2 to 60 rpm, preferably 10 to 20 rpm, e.g. 15 rpm.

Alternatively, the separation surface may slowly rotate in the liquid aliquot containing the retentate from the filtering step or may be placed in a rotating tube filled with said aliquot. Yet another option is (if necessary repeatedly) flowing the sample over a flat, optionally tilted (e.g. about 30° to 40°, preferably about 35°) separation surface, e.g. a coated slide. In such an example, the surface of the slide can be partially or completely submerged in a suitable physiological buffer (see, for example, the buffers defined above in the context with sample dilution). As the as the cells in the sample have a higher specific gravity than the buffer, they will flow along the tilted separation surface into the direction of the bottom of the device.

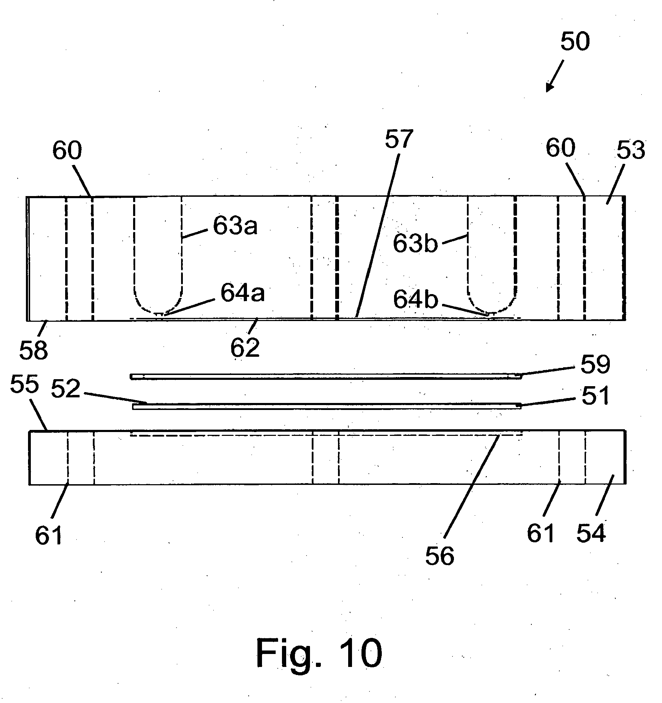

In an even more preferred example, the incubation is carried out in a flow-through device, for example, a device as depicted in figure 10.

In the case of coated particles, the particle suspension is mixed with the purified sample and gently agitated, as for example in a closed tube on a rotation incubator at low speed.

After the cells and the separation surface have been incubated under conditions which allow for the binding of the affinity molecules to the target cells, any unbound cells and unbound materials are removed. This is achieved by separating the separation surface from such unbound cells and materials. Preferably, this step also includes washing the separation surface with a suitable washing buffer, such as the iso-osmolaric buffers mentioned above in the context with sample dilution.

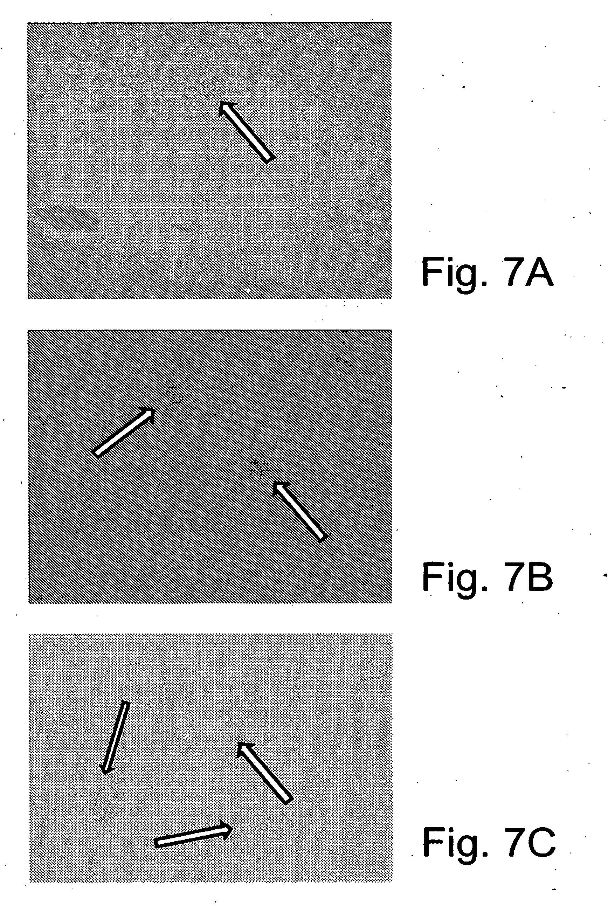

It is possible to isolate target cells in a native state which allows for the subsequent downstream processing of the cells. For example, the cells isolated by the method described above can be cultured in standard cell culturing media. This is, for example, a particular advantage with tumor cells which can be further characterized after isolation. As a further advantage, the cells obtained by the method disclosed herein show an unimpaired cell morphology compared to cells isolated according to methods known in the prior art. As shown in figure 7, cells isolated with common magnetic particle-based systems (e.g. Veridex) tend to lose their spherical shape. In consequence many cells will be not identified due to a loss of the nucleus, a unregular plasma-nucleus ratio and the occurrence of cell debris which stains positively for markers for antibody detection thus leading to false positive results. In contrast, the spherical morphology of tumor cells captured by the method of the invention is not altered due to the fact that the cells are not coupled to any sharp-edged particles and are bound to a soft surface floating on the top of macromolecules.

In a further aspect, the invention relates to a method for the detection and/or quantification of target cells in a sample, which sample comprises red blood cells and/or platelets. The method comprises
a) performing a method as described above in the context with the isolation and/or enrichment of the target cells; and
b) detecting and/or quantifying the target cells by suitable means.

Methods for detecting target cells that have been captured are well known in the prior art. For example, these methods can be based on the detection of marker molecules, e.g. surface protein or saccharide structures or nucleotide sequences, which are highly specific for the target cells to be detected and/or quantified. The choice of suitable marker molecules will depend on the particular target cell, and the skilled person will have no problems to select appropriate marker molecules and corresponding affinity molecules which specifically bind to these marker molecules.

According to a further aspect, the detection and/or quantification of the target cells can be performed by use of detectably labelled affinity molecules, such as detectably labelled antibodies or antibody fragments. The affinity molecules to be used in the detection and/or quantification step can be, for example, the same molecules that have been described elsewhere herein for immobilization on the separation surfaces. For example, antibodies or fragments of antibodies can be used which are either labelled or which can be detected themselves by labelled secondary antibodies.

According to another aspect, the detection and/or quantification of the target cells involves detectably labelled DNA probes. More preferably, the detection and/or quantification comprise fluorescent in-situ hybridization (FISH). Various protocols for conducting a FISH analysis using labelled probes of different length in intact target cells are available in the art (for example, detection of egfr amplification using specific DNA probes). In an in-situ hybridization assay, the target cells to be detected and/or quantified are typically permeabilized and the DNA of the cells is melted partially. The DNA is then contacted with a hybridization solution at a moderate temperature to permit annealing of fluorescently labelled probes specific for the particular nucleic acid sequence selected for detecting the target cells. For example, when the detection and/or quantification of tumor cells originating from an epithelial tissue in the blood is desired, a labelled nucleic acid probe directed to egfr, HER2, PI3-kinase, c-myc, akt, and the like may be used. The probe which has been hybridized to the DNA of tumor cells, shows a number of egfr spots greater than 2, for example 3, 4, 5, 6, 7, 8, 10 or more and can be distinguished from DNA of leukocytes that may have adsorbed to the separation surface (2 spots at most). FISH probes are typically labelled with one or more fluorescent reporters. After hybridization with the probe, the target cells are washed at a predetermined stringency or at an increasing stringency until an appropriate signal to noise ratio is obtained. The hybridized probes are then monitored, e.g. by fluorescence microscope. By using multiple nucleic acid probes with different fluorescence colors, a multicolored analysis (i.e., for different sequences) can be performed in a single step on the separation surface. The nucleic acid probes used in FISH assays are usually longer than those used, for example, in Southern-blotting. FISH probes may have a size of about 1, 5, 10, 20, 30, 40, 50, 60 or up to 100 kb, or even of 200, 300 or 400 kb. FISH probes may be directly labelled (e.g. by use of fluorescent dyes) or indirectly labelled (e.g. by a hapten, such as digoxigenin or biotin). It is preferred to use fluorescent labels, so that the result of the hybridization to the genomic DNA of the test sample (e.g. cells of tissue derived from biopsy) can directly be observed. Labeling kits for fluorescence labeling may be obtained from different manufacturers, such as the labeling kits SpectrumOrange, SpectrumGreen, and SpectrumRed purchasable by Vysis Inc., Downer's Grove, Illinois, USA.

It is of course also possible to combine one or more of the above methods for detecting and/or quantifying target cells in order to improve the sensitivity. For example, the detection and/or quantification can be based on a combined method of fluorescent in-situ hybridization and antibody-based detection, as described below in the examples.

According to one aspect, the detection and/or quantification of the target cells involve a PCR, preferably a real-time PCR. In this approach, primers directed to marker genes, which are common for the respective target cells, are used to amplify products that indicate the abundance of these cells in a sample. For example, if the target cell is a tumor cell, such as a dissiminated tumor cell, one or more genes encoding the specific cell surface marker discussed above can be used as templates for PCR amplification. For a dissiminated tumor cell, suitable genes encoding surface markers are those which encode, for example, the epidermal growth factor receptor (EGFR), the epithelial cell adhesion molecule (EpCAM or CD326), the insulin growth factor-1 receptor (IGF-1 R), the epidermal growth factor receptor 2 (Her2), cytokeratin 19 (CK19), cytokeratin 20 (CK20), mucin 1 (MUC1), mucin 2 (MUC2), and the like. In a particularly preferred aspect, a combination of primer directed to different marker genes is used for the detection and/or quantification of target cells.

Moreover detection of tumor cells can be achieved using two-colour photoacoustic methods. Thereby, the use of gold-plated carbon nanotubes as a second contrast agent allows multiplex detection (see, for example, Galanzha et al. (2009), Nat. Nanotechnol. 4, 855).

In the following, exemplary advantageous aspects of preferred embodiments of the invention are explained in more detail with reference to the figures.

### BRIEF DESCRIPTION OF THE FIGURES

Fig. 1 shows the incubation of EGFR-expressing breast tumor cell line MDA-MB-468, which are present in human serum, on a partially antibody derivatized hydrogel surface. Left: Anti-EGFR-IgG functionalized hydrogel surface. Upper right corner: blank hydrogel surface (control).
Fig. 2 shows a comparison of non-specific binding (NSB) from leukocytes from different preparations on varying polycarboxylate (HC)-surfaces (with and without antibody). Leukocytes fractions were prepared from whole blood by a) density gradient centrifugation or b), c) by cell sieving. Incubation was done in 2 ml PBS containing 0.96 to 1.15 million cells in Cytospin centrifugation tubes with EpCAM modified HCX-Slides (XanTec bioanalytics GmbH, . Dusseldorf, Germany) and non-reactive HC-slides (XanTec bioanalytics GmbH, Dusseldorf, Germany).

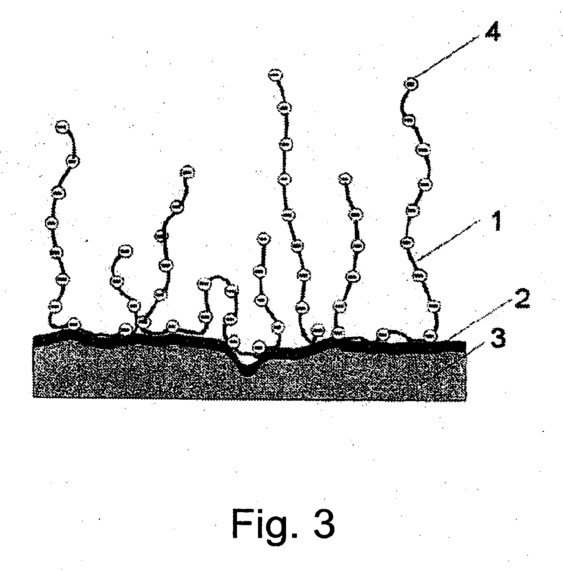

Fig. 3 shows a schematic depiction of a hydrogel coating that can preferably be used in the method of the invention. The substrate (3) is coated with an adhesion promoting layer (2) and a hydrophilic polymer (1), the latter of which has a brush-like structure. The hydrophilic polymer (1) is arranged vertically to the substrate surface, so that a multiplicity of functional groups (4) is available for the immobilization of the affinity molecules.
Fig. 4 shows a schematic illustration of an embodiment of ari arrangement for performing the filtration step.
Fig. 5 shows a schematic illustration of another embodiment of an arrangement for performing the filtration step.
Fig. 6 shows the recovery rate of MDA-468 tumor cells.after affinity separation using an antibody-modified hydrogel slide.
Fig. 7 shows a comparison of cell morphology after different cell isolation processes; A & B: tumor cells captured by the Veridex system are surrounded by anti-EpCAM antibody coated magnetic particles-and aggregated platelets. Spherical morphology of captured cells is lost; C: tumor cells captured on anti-EpCAM antibody coated HC-Slides.
Fig. 8 shows a schematic illustration of yet another embodiment of an arrangement for performing the filtration step.
Fig. 9a illustrates a first method of performing the filtration step utilising the arrangement of Figure 8.
Figs. 9b and 9c illustrate a second method of performing the filtration step utilising the arrangement of Figure 8.
Fig. 10 shows a schematic illustration of an embodiment of an arrangement for performing the separation step.
Figs. 11 a and 11 b show a schematic illustration of a further embodiment of an arrangement for performing the separation step.

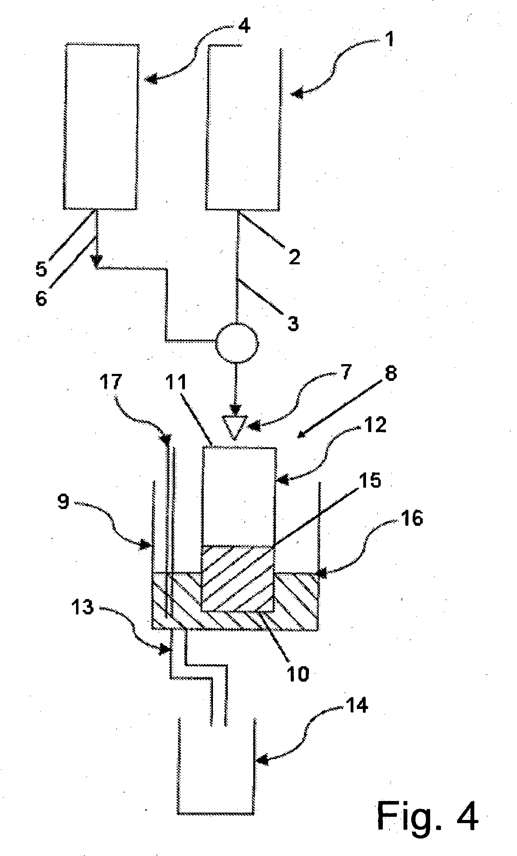

In Fig. 4, an arrangement for performing the filtration step comprises a sample reservoir 1 for receiving and containing the diluted or undiluted sample possibly including the target cells. The reservoir 1 has an outlet aperture 2 through which, in operation, the sample exits the reservoir 1 and enters a conduit 3. As shown in Fig. 4, the outlet aperture 2 is preferably disposed in the bottom of the reservoir 1 and the reservoir 1 and the conduit 3 are preferably arranged such that the delivery of the sample from the reservoir 1 to the conduit 3 and the movement of the sample within the conduit 3 are effected or at least assisted by gravity. Throughout the present description of the figures, terms such as "bottom" and "top" relate to the orientation shown in the figures.

In addition to the reservoir 1 a further reservoir 4 may optionally be provided for receiving and containing a solution or buffer that may serve to dilute the sample. The reservoir 4 has an outlet aperture 5 through which, in operation, the solution exits the reservoir 4 and enters a conduit 6. Again, the outlet aperture 5 is preferably disposed in the bottom of the reservoir 4 and the reservoir 4 and the conduit 6 are preferably arranged such that the delivery of the sample from the reservoir 4 to the conduit 6 and the movement of the sample within the conduit 6 are effected or at least assisted by gravity. The conduit 6 is connected to the conduit 3 such that the sample and the solution may be mixed.

The delivery end 7 of the conduit 3 is arranged such that the sample, optionally mixed with the solution from the reservoir 4, can be supplied continuously, discontinuously or intermittently to a generally cylindrical filter component 8 that is disposed within a container 9.

The filter component 8 comprises at its bottom longitudinal end a plate 10 and at its upper longitudinal end a ring 11. The plate 10 and the ring 11 are interconnected by a plurality of longitudinal rods (not shown in the figures). A sheet-shaped filter element 12 is disposed annularly closed around the arrangement of rods such that it forms the circumferential surface of the cylindrical filter component 8. In this regard, it is also possible that the sheet-shaped filter element 12 is disposed annularly closed inside the space defined by the rods by attaching it to the radial inner face of each of the rods. The plate 10 and the ring 11 may have e.g. a circular, oval, square, rectangular, hexagonal or other shape, with the cylindrical filter component 8 having a corresponding cross-sectional shape.

By means of this construction the sample fed from the delivery end 7 of the conduit 3 can enter the filter component 8 through the opening of the ring 11, and filtrate exits the filter component 8 through the filter element 12 and is collected within the container 9. A further conduit 13 is provided, e.g. connected to the bottom of the container 9, through which conduit 13 the filtrate may be removed from the container 9, e.g. by draining under the influence of gravity or by utilising suitable pumps or suction devices. A further container 14 is provided for collecting the filtrate removed from the container 9.

In operation, the flow of sample into the filter component 8 and/or the rate of removal of filtrate from the container 9 are controlled such that the absolute level of the surface 15 of the liquid within the filter component 8 is higher than the level of the surface 16 of the liquid within the container 9. In this manner, a difference in hydrostatic pressure is advantageously maintained on both sides of the filter element 12 that forces the filtrate through the filter element 12.

A gas conduit 17 may optionally be provided for introducing gas into the filtrate for advantageously generating a movement within the liquid.

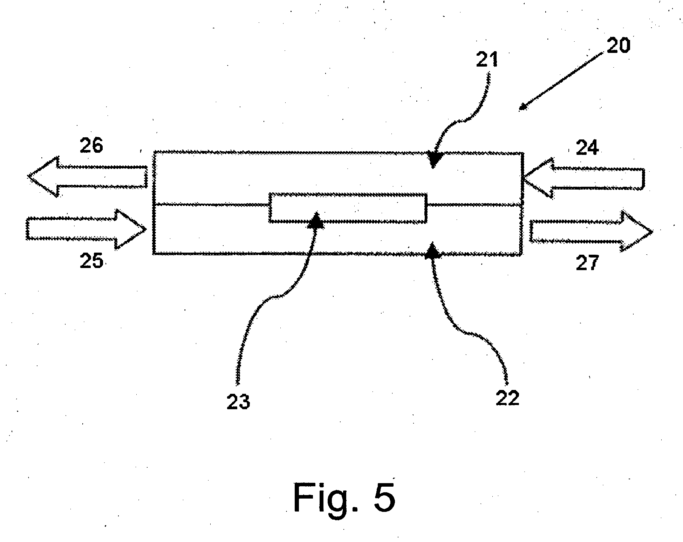

In an alternative embodiment schematically shown in Figure 5 an arrangement for performing the filtration step is a cross-flow filtration arrangement 20. In the usual manner, it comprises two conduits 21, 22 that are extending parallel to and in contact with each other, and a filter element 23 is arranged in aligned openings in the side walls of the conduits 21, 22 such that liquid is able to pass via the filter element 23 between the two conduits 21, 22. In operation, a flow 24 of the diluted or undiluted sample is introduced into one end of conduit 21 such that it is passed tangentially across the filter element 23. An oppositely directed flow 25 of a physiological medium is introduced into the opposite end of the conduit 22 such that it is passed tangentially across the opposite face of the filter element 23. By maintaining a higher pressure within the conduit 21, the filtrate is forced through the filter element 23 into the conduit 22 such that retentate 26 exits the conduit 21 and a mixture 27 of the filtrate arid the physiological medium exits the conduit 22.

A further alternative embodiment of an arrangement for performing the filtration step is schematically shown in Figure 8. This arrangement 30 comprises two bodies 31 and 32 which, in the illustrated embodiment, have a cuboidal shape. The bodies 31 and 32 are disposed such that surfaces 38a and 38b of the body 31 and the body 32, respectively, extended parallel to and at a distance from each other. In this manner, a gap or spacing 39 is present between the two bodies 31 and 32.

In each of the surfaces 38a and 38b, a recess 31 a and 32a, respectively, is formed in the respective body 31 and 32, respectively. In the illustrated embodiment the recesses 31a and 32a are likewise of a cuboidal shape. The openings of the recesses 31a and 32a, which face each other, are closed by a common filter element 33 suitably disposed in the spacing 39 between the bodies 31 and 32. In order to ensure a sealing closure of the recesses 31 a and 32a, the filter element 33 may be carried in a frame 33a having an annular construction and being provided with suitable sealing elements, which sealing elements sealingly engage the surfaces 38a and 38b and surround the openings of the recesses 31a and 32a.

Two conduits 34 and 36 extend from the recess 31 a and project from the surface 40a opposite the surface 38a, and two conduits 35 and 37 extend from the recess 32a and project from the surface 40b opposite the surface 38b, and these conduits 34, 35, 36 and 37 allow to introduce fluids into and remove them from the interior of the recesses 31a and 32a. Thus, it is in principle possible by means of this arrangement 30 to perform a cross-flow filtration similar to the case described with reference to Figure 5 by suitably introducing a flow of the diluted or undiluted sample through e.g. the conduit 34 and a flow of a physiological medium through e.g. the conduit 37.

However, the arrangement 30 shown Figure 8 is particularly advantageous for performing two different methods of filtration.

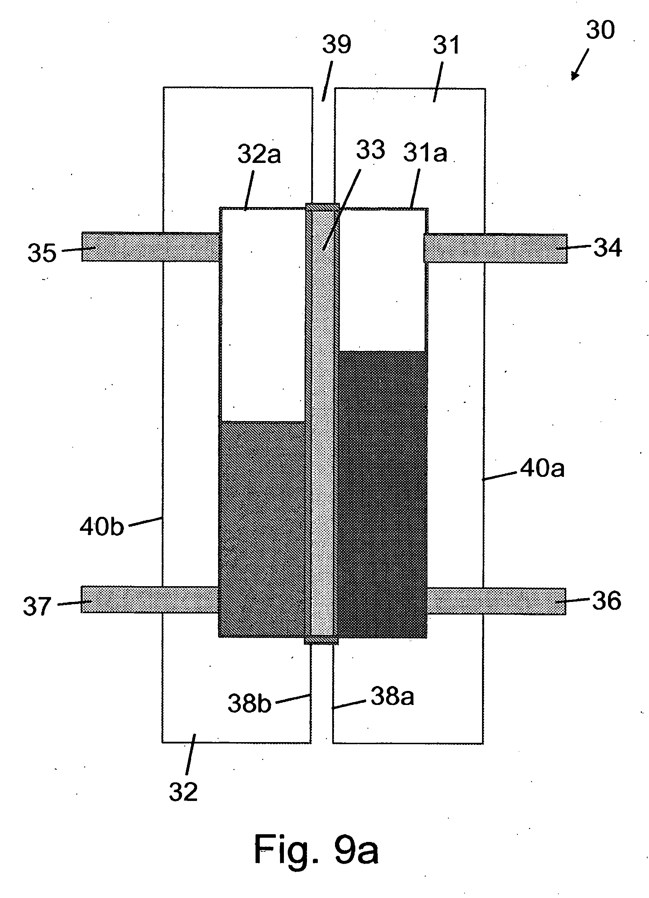

The first method, in which the arrangement 30 is not moved and is oriented such that the filter element 33 and the spacing 39 extend in a vertical plane, is illustrated in Figure 9a. For an effective filtration both recesses 31 a and 32a are at first filled with buffer to a level of about 50% of the interior recess volume. Subsequently, the diluted or undiluted sample is introduced through e.g. the conduit 35 into the recess 32a, thereby raising the level of liquid within the recess 32a as compared to the level of liquid within the recess 31a. In this manner, a difference in hydrostatic pressure is created on both sides of the filter element 33 that forces the filtrate through the filter element 33. Of course, it is also possible to immediately fill the recess 32a to a suitable level with a mixture of buffer and sample.

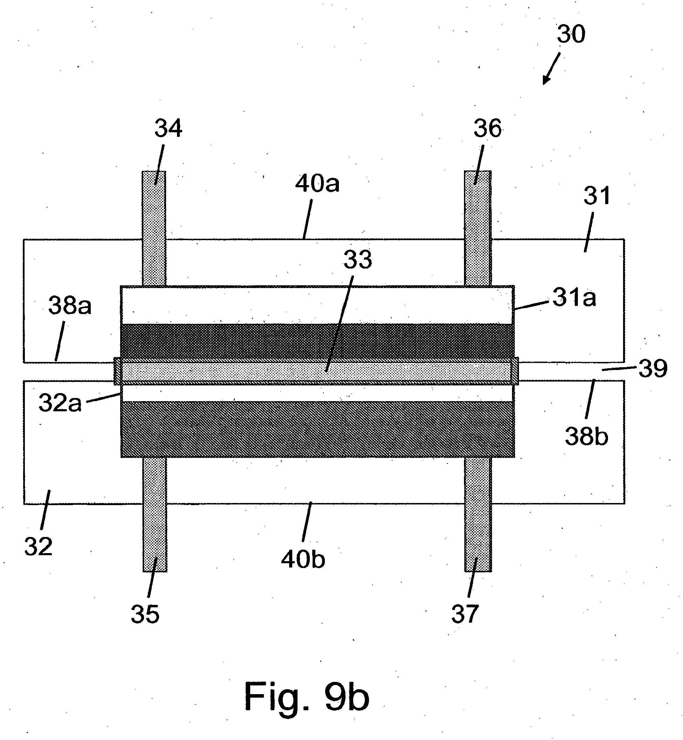

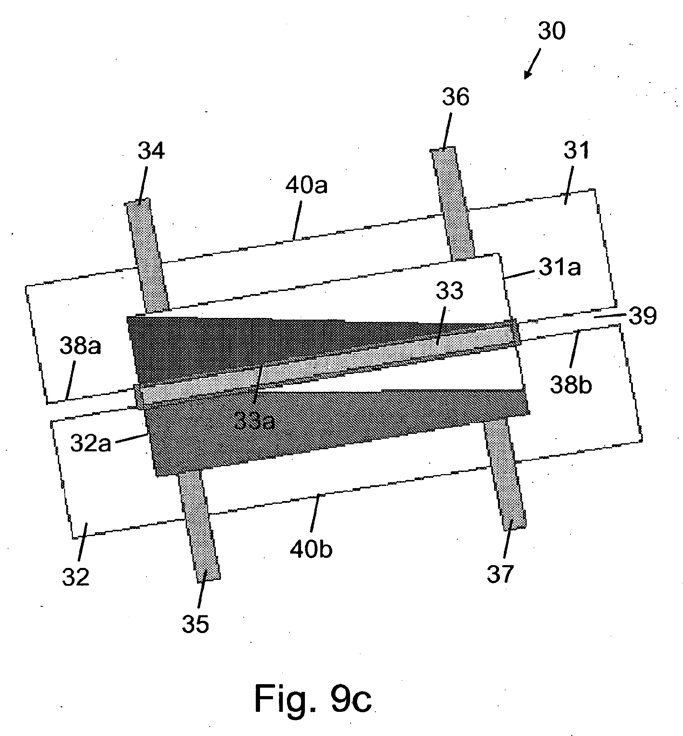

The second method, in which the arrangement 30 performs a rocking motion and is initially oriented such that the filter element 33 and the spacing 39 extend in a horizontal plane, is illustrated in Figures 9b and 9c. At first, both recesses 31a and 32a are again filled with buffer to a level of about 50% of the interior recess volume. Subsequently, the diluted or undiluted sample is introduced through e.g. the conduit 35 into the recess 32a. Starting from the horizontal orientation shown in Figure 9b, the arrangement 30 is put into a, preferably slow, rocking motion by alternately tilting the arrangement 30 in two opposite directions, as illustrated in Figure 9c. The preferred maximum tilt angle is 25°. In the tilted positions assumed in the course of the rocking motion of the arrangement 30, the liquid volumes in the two recesses 31a and 32a contact each other through portions of the filter element 33. Due to the rocking motion, the area of contact travels across the entire surface area of the filter element 33. The rocking motion may advantageously be effected by means of a rocking shaker.

Figure 10 shows a cross-sectional view of an arrangement 50 for contacting the retentate produced by means of the filtration step with a separation surface. The arrangement 50 comprises a, preferably rectangular, slide 51 having the separation surface 52.

Further, the arrangement 50 comprises two bodies 53 and 54 which, in the illustrated embodiment, have a cuboidal shape. In the upper surface 55 of the lower body 54 a recess 56 is provided which is shaped and sized to receive the slide 51 together with an annular sealing element 59 and such that at least the sealing element 59 slightly projects from the surface 55. A corresponding recess 57 is provided in the lower surface 58 of the upper body 53. The latter recess 57 is sized and shaped to matingly receive the portion of the sealing ele- . ment 59 projecting from the surface 55 of the lower body 54 when the two bodies 53 and 54 are attached to each other with the two surfaces 55 and 58 being adjacent and extending parallel to each other as will be explained in more detail below.

In operation the slide 51 is positioned within the recess 56, and the annular sealing element 59 is positioned on top of the slide 51. This sealing element 59 is dimensioned and shaped such that it circumferentially engages the upper separation surface 52 of the slide 51 in an edge region thereof. Then, the body 53 is placed on top of the body 54 in such a manner that the annular sealing element 59 is received within the recess 57, and the two bodies 53 and 54 are fixedly secured to each other by means of e.g. screws that are introduced through a plurality of corresponding bores 60 and 61 in the bodies 53 and 54, respectively. The recesses 56 and 57 are dimensioned such that in this position the separation surface 52 of the slide 51 is spaced 3 to 500 µm from the interior wall 62 of the recess 57.

In the upper body 53 two through bores are provided having large diameter sections 63a and 63b, respectively, and small diameter section 64a and 64b, respectively. The small diameter sections 64a and 64b open into the recess 57. Thus, it is possible in operation to introduce the retentate through the through bore 63a, 64a into the recess 57 at one end thereof, and to remove the filtrate from the recess 57 at the opposite end via the through bore 63b, 64b, with the retentate flowing along and in contact with the separation surface 52. Due to the narrow spacing between the separation surface 52 and the interior wall 62 of the recess 57, the flow is essentially and advantageously a laminar flow.

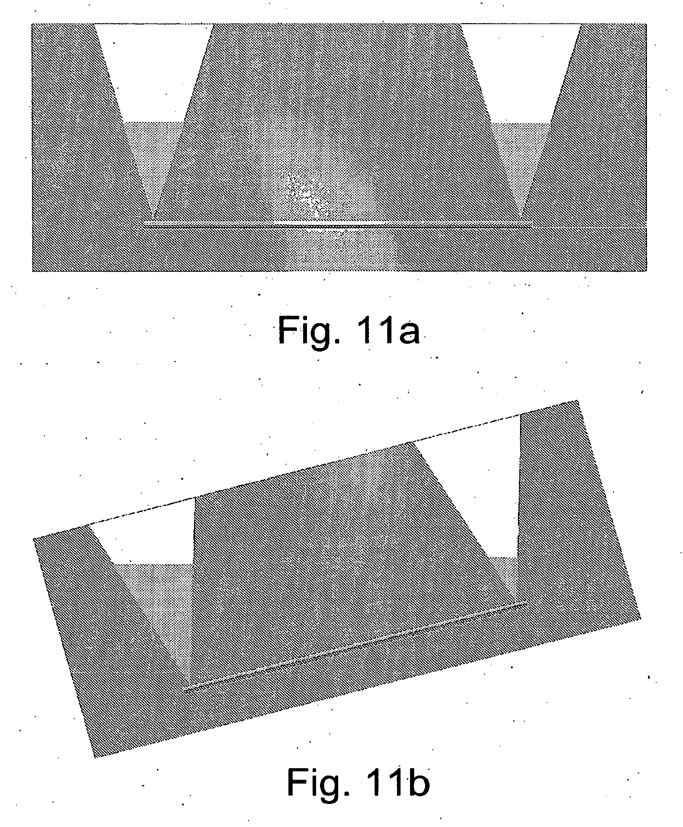

Also, it is possible to simultaneously fill the retentate into both through bores 63a, 63b and 64a, 64b and to put the arrangement into a rocking motion as schematically shown in Figures 11a and 11b. In these. Figures, the through bores 63a, 63b and 64a, 64b have a conical shape in order to reduce the dead volume.

### EXAMPLES

### Example 1

### Preparation of a test sample containinqtumor cells

Cultured MDA-468 cells derived from human breast carcinoma were pre-stained with 4',6-Diamidino-2-phenylindol (DAPI, Pierce/ThermoScientific) without permeabilization., DAPI is a cationic fluorescent dye which binds to adenine-thymine-rich DNA. DAPI is regularly used for staining cell nuclei. The stained cells can be counted by visual inspection or by suitable automated devices. The cells were washed twice with Dulbecco's modified Eagle Medium (DMEM) to remove excessive dye. Each suspension of pre-stained cells was controlled for fluorescence emission. Aliquots of tumor cells were counted manually and spiked in human whole blood samples collected from healthy donors by venipuncture in collection tubes with EDTA to prevent coagulation. The blood samples were used within'4 h from collection. For recovery testing, the tumor cells were diluted to cell numbers between 50 and 500 cells/100 µl to minimize dilution and counting errors.

### Example 2

### Preparation of a woven fabric for cell sieving

The filter of a blood administration set (Sarstedt AG & Co; 74.4255) was separated and the woven filter removed. A sheet of a woven fabric (Sefar AG, Sefar Nitex 03-5/1, Sefar Petex 07-5/1) having a mesh size of 1 µm was fixed on the filter rack by use of an adhesive. After fixing the woven fabric to the filter rack, any remaining solvent deriving from the adhesive was removed in a vacuum for 30 min. A leak test was carried out with a cell suspension of monocytes from buffy coat obtained by centrifugation of 500 ml blood over the whole volume of the filter rack. The so prepared filter rack was fixed in the middle of a beaker by using a double-faced medical adhesive (50 µm thick) so that the closed bottom of the filter rack was in contact with the bottom of the beaker.

### Example 3

### Removal of red blood cells and platelets from the sample

A pre-wetting of the filter rack prepared in example 2 is preferred to prevent initial unspecific adhesion of cells to the filter unit. Hence, a volume of z10-15 ml of a physiological and isoosmolaric buffer (e.g. PBS) was added to the filter rack. The filtering was performed in an assembly as depicted in Figure 4. The buffer was applied.to the center of the cylindrical filter rack by a conduit which is located 1-2 cm above the opening of the filter rack. A flow rate of 1 ml/min was set with a peristaltic pump. A blood sample as prepared in example 1 (stored for 24 h under agitation at room temperature) containing between 10 and 120 MDA-468 cells per ml in spike-in experiments was applied to the filter rack by a second conduit with a flow rate of 100-200 µl/min. In this way, dilutions of the blood samples of in a ratio of about 1:5 to-1:10 could be obtained.

Due to the narrow mesh size of the woven filter element, the liquid meniscus in the inner compartment of the filter rack was higher than the meniscus in the beaker enclosing the filter rack (see Figure 4). The resulting pressure led to the removal of red blood cells and platelets which passed the meshes of the woven filter element and got enriched in the beaker. To maintain the meniscus in the beaker, a waste conduit was fixed at the bottom of the beaker which was connected with a peristaltic pump. The flow rate of the waste conduit was calculated as the sum of the flow rates from the sample conduit and the buffer conduit. After a predetermined volume of a whole blood sample had been applied, buffer addition to the filter rack was stopped. Due to the continuous draining of buffer by the waste conduit, the liquid volume in the filter element likewise decreased. The drain off was stopped at a volume of about 1-3 ml cell suspension that was left in the filter element. The retentate cell suspension was then pipetted into a reaction tube. To assure that all cells in the filter device were transferred to the incubation chamber, the inner face of the filter element was washed five times with 2 ml PBS and then pipetted into the tube. The tube was spun down at 800 rpm for 2 min. The supernatant of 9 ml was discarded and the cell pellet with the residual volume was resuspended in 1 ml PBS for subsequent transfer to a hydrogel slide.

### Example 4

### Derivatization of a hydrogel-coated slide for planning

A pre-activated polycarboxylate hydrogel slide (SL-HCX-5, XanTec Bioanalytics GmbH, Dusseldorf, Germany) was covered with 20 µl of a 250 µg/ml anti-EpCAM/Trop-1-antibody solution (AF960; RnD Systems, Minneapolis, USA). Prior to coupling, the antibody was dialyzed against a 5 mM sodium acetate solution (pH 5.0). The antibody solution was dried at ambient conditions (room temperature and relative humidity of 50-75%), and the solution was further incubated for additional 20 min at said conditions after drying. In the next step, the whole slide was placed in a Petri dish and covered with 1 M ethanolamine solution, pH 8.5, under gentle shaking for 20 min. Two washing steps were carried out for 10 min each under agitation in the Petri dish using phosphate-buffered saline (PBS) to eliminate residual ethanolamine. For subsequent storage, the slide was rinsed with distilled water and dried in a sharp stream of nitrogen. The slides prepared in this manner were ready for incubation with the blood-derived cell suspensions obtained from examples 1-3 containing the MDA-468 cells.

Alternatively, in order to provide a hydrogel-coated slide for panning with a particularly low background of unspecifically attached non-cancer cells (e.g. blood leukocytes), a hydrogel slide (SL-HC-5, XanTec Bioanalytics GmbH, Dusseldorf, Germany) was placed in a Petri dish or an appropriate slide rack. An activation solution of 0.5 M MES, 0.05 M NHS with 0.01-0.025 % (w/v) EDC was prepared immediately before use. The hydrogel slides were covered with this solution and incubated for 5 min with gently shaking followed by an incubation in 2 mM acetic acid for at least 30 sec. Then the slides were rinsed with distilled water and either dried in a sharp stream of nitrogen or by centrifugation. Slides made accordingly to this activation procedure have a lower amount of reactive succinimidyl ester formed in the hydrogel compared to the above mentioned HCX slides and therefore a lower immobilization capacity for biomolecules (for example, antibodies) which decreases the background of non-specifically bound leukocytes during incubation. Modification of the hydrogel with antibodies can be performed as indicated above in connection with the SL-HCX-5 slide.

### Example 5

### Incubation of tumor cells on hydrogel surfaces

2 ml of the MDA-468 cell suspension obtained in above Example 3 were then transferred into the incubation chamber of a cyto-system (Hettich AG, Germany) with an antibody-coated slide (SL-HCX-5, XanTec bioanalytics, Dusseldorf, Germany) already mounted in the cyto-system A rocking platform shaker (Heidolph Duomax 1030) with a tilt angle of 5° was adjusted to 2 cycles/min. Cells were incubated over 4 hours at room temperature. After the incubation, the supernatant was discarded. In the following washing step, 2 ml PBS were pipetted into the incubation chamber. After incubation for 4 hours at room temperature, the PBS buffer was discarded and washing was repeated once.

### Example 6

### Deterrriination of the recovery rate after sieving

15 ml of blood drawn from a healthy volunteer as described in example 1 was divided in 5 ml-aliquots. 110 (+/-2) MDA-468 cells were spiked into each aliquot. Sieving was carried out with each of the three aliquots as described in example 3. After sieving, a residual volume of approx. 2 ml cell suspension was left in the filter rack. The filter rack containing the cell suspension was then carefully removed from the beaker and transferred in a cyto-spin system (Hettich) which was prepared according to the manufacturer's guidelines using a super frost glass slide, so that the openings of the filter rack were oriented in the direction of the glass slide. Detection of pre-stained tumor cells was carried out by fluorescence microscopy manually and automated (Ariol Platform. Genetix Ltd. New Milton, Hampshire, UK. Recovery rates from 99 to 100% were reached (see table 1 below).

**Table 1: Recovery rate after sieving**

| | cell numbers (manual counting) | cell numbers (automatic counting) |
|---|---|---|
| aliquot 1 | 109 | 111 |
| aliquot 2 | 110 | 111 |
| aliquot 3 | 109 | 109 |

### Example 7

### Recovery rate in the affinity based cell separation with antibody modified hydrogel slide

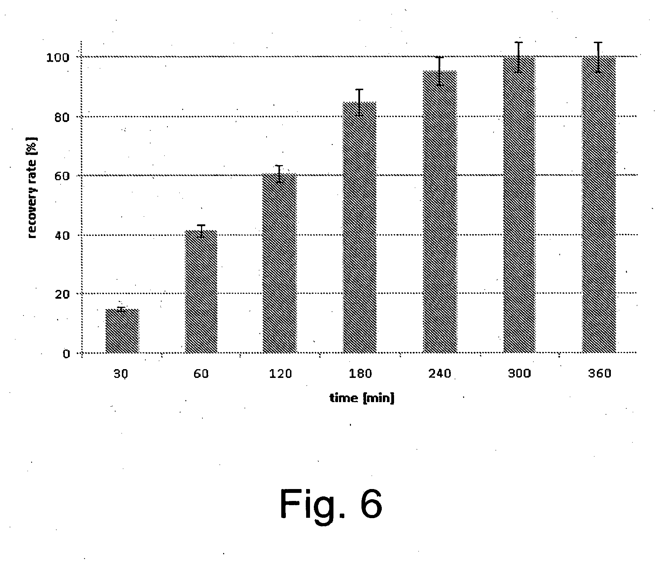

75 ml of whole blood were drawn from a healthy volunteer as described in example 1, and the complete sample was subjected to the sieving step described in example 3. After,_sieving a residual volume of approx. 3 ml of cell suspension was left in the filter rack. The cell suspension was diluted to a volume of 30 ml with PBS, and 14 aliquots of 2 ml were pipetted into cytospin tubes (Hettich) which had been prepared with antibody coated HC-hydrogel slides instead of frosted glass slides according to the manufacturer's protocol. Immediately after transfer of the cell suspensions into the cytospin tubes, 120 (+/-2) MDA-468 cells that had been pre-stained with DAPI were spiked into the aliquots. Incubation was carried out on a rocking platform shaker (Heidolph Duomax 1030) at a speed of 2 cycles per minute at room temperature. Cell counts were determined after 30, 60, 120, 180, 240, 300, and 360 min incubation on the hydrogel surface by manual .cell counting in a fluorescence microscope (Leica DMLB, Leica Microsystems GmbH, Wetzlar, Germany) for DAPI pre-stained MDA-468 cells. The cell counts as measured in parallel samplers are depicted in the below table 2. A time course of recovery rates is shown in fig. 6.

**Table 2: Recovery rate after hydrogel slide separation**

| Tirne (min) | Count 1 | Count 2 | Mean value (%) |
|---|---|---|---|
| 30 | 20 | 15 | 14,53 |
| 60 | 47 | 52 | 41,25 |
| 120 | 78 | 67 | 60,42 |
| 180 | 95 | 108 | 84,58 |
| 240 | 117 | 111 | 95 |
| 300 | 121 | 118 | 99,58 |
| 360 | 123 | 116 | 99,58 |

### Example 8

### Detection of MDA-468 cells by FISH and immunocytochemistry

MDA-468 cells which have been isolated by use of a hydrogel slide as described in example 5 were detected by a combined protocol for FISH and immunocytochemistry. This approach was focussed on the detection of chromosomal aberrations and tumor-specific gene expression. For the detection of gene amplifications of the egfr gene by FISH, probes complementary to the sequence of the egfr gene were prepared from BAC DNA (clones RP5-10191 E12) with the usage of Large DNA Construct Isolation Kit (Qiagen, Germany) and BioPrime@ Total Genomic Labelling System (Gibcolnvitrogen, UK) according to the manufacturers' protocols.

The MDA-468 cells were fixed on hydrogel slides in the incubation chamber of a cyto-system (Hettich AG, Germany) (in ice-cold 75% ethanol for 2 min and pre-treated with 100 µg/ml RNase A for 40 min at room temperature. Cells were then treated in 1x citrate buffer (pH 6.0, Dako, Denmark) for 3 min at 120°C. The cells were again fixed in 1% formaldehyde in 1 x PBS for 10 min and dehydrated in a series of alcohols. Air-dried cells were denatured in denaturation buffer (70% formamide, 0.6 x SSC, pH 7.4) for 5 min at 73°C and dehydrated in a series of alcohols. 1 µl of Cot Human DNA (Roche, Germany), 0.5 µl of CEP7 Spectrum Aqua (Abbott Molecular) as a reference probe and 2 µl of spectrum orange-labelled probe for the human egfr gene suspended in 6.5 µl of hybridization buffer (Abbott Molecular) were applied on the top of the slide. The specimens were denatured for 3 min at 95°C and hybridized for at least 16 hours at 37°C. Afterwards, they were washed 2 min in 2 x SSC/NP-40 buffer at 72°C and in room temperature (RT). The next day, the slide was washed 3 times for 10 min in 50% formamide/2 x SSC buffer, 2 times for 10 min in 2 x SSC buffer and 5 min in 0.1 x SSC/0.1% Tween-20 buffer at 46°C. The cells were permeabilized 3 times for 3 min in 1 x TBST. The non-specific binding was reduced by 20 min incubation in Blocking Serum (Dako, Denmark).

The primary mouse monoclonal antibodies against pan-cytokeratin (A45, Mikromet, Germany) or vimentin (DB Pharmingen^{™}) diluted 1:100 or 1:200, respectively, in DakoREAL^{™} Antibody diluent (Dako, Denmark) were incubated with the cells for 45 min at room temperature. The specimen was then incubated with a secondary Alexa-488-labelled anti-mouse antibody (MiBioTech) diluted 1:200 for 45 min at room temperature. Finally, the slide was washed 2 times for 3 min with 1xTBST and once for 3 min with 1xPBS, and counterstained with Vectashield®O Mounting Medium with DAPI (Vector Laboratories Inc.).

The cytokeratin- or vimentin-positive cells, showing no apoptotic or necrotic morphology, were analysed for their egfr gene dosage. Additionally, a total of 500 cytokeratin-positive and vimentin-positive cells were scored for the frequency of egfr gains and compared to the score obtained in 3 healthy volunteers. The specimens were analysed under a fluorescence microscope (MetaSystem GmbH, Zeiss, Germany). For each cell, the ratio between target and reference probe was counted and the mean counts from 20 cells was taken for evaluation, containing minimum 2 signals for reference the CEP7 probe. It could be shown that MDA-468 cells harboured from 3 to 7 spots for the fluorophor-labelled EGFR probe and simultaneously 2 spots for the CEP7 reference probe.

### Example 9

### Whole genome amplification

In an alternative approach, MDA-468 cells on a hydrogel surface were detected by PCR. In a first step, the MDA-468 cells on the hydrogel surface were cooled to 0°C on ice, overlaid with chilled 9 µl of the Genomiphi sample buffer (GE Healthcare). Single-cell transfer to PCR reaction tubes was performed by using the micromanipulator CellTram vario equipped with the custom-made capillary Kappa MFKII coupled with a TransferMan NK 2 (Eppendorf) attached to the inverted microscope Axiovert 200 (Zeiss). The tubes were kept at -80°C for a minimum time of 15 min. After thawing 1 µl of a 1:10 protease solution (Qiagen) was added to each tube. The cells were then incubated in a thermocycler at 50°C (15 min) for digestion and at 70°C (15 min.) for enzyme inactivation. To amplify the genome of the tumor cells, whole genome amplification (WGA) was performed using the Genomiphi Kit (GE Healthcare) according to the manufacturer's instructions. After the WGA reaction remaining dNTPs and random hexamer primer were removed from the product using the NucleoSEQ kit (Macherey-Nagel) according to the manufacturer's instructions. The samples prepared in this way were used in the amplification of the different target sequences described below.

### Example 10

### Allelic imbalance (Al) analysis

The allelic imbalance (Al) analysis is based on the detection of length polymorphic sequences comprising simple sequence repeats of cytidin and adenosine (microsatellite). An allelic imbalance is determined if the ratio of peak areas in a capillary electrophoretic evaluation between allele 1 and allele 2, differing in the number of CA repeats, exceeds 1.3 or undercuts 0.7._ PCR reactions were performed on a Mastercycler Gradient (Eppendorf) in a total reaction volume of 10 µl, containing 20 ng of template DNA from MDA-468 tumor cells (genomic DNA or WGA product from example 9), the primers depicted in SEQ ID NO:1 and SEQ ID NO:2 (SEQ ID. NO:1, CA-SSR1_For: [6-FAM]GTTTGAAGAATTTGAGCCAACC; SEQ ID NO:2, CA-SSR1_Rev: TTCTGTCTGCACACTTGGCAC), deoxynucleotide triphosphate, AmpliTaq Gold (Applied Biosystems), TMAC, MgCl₂ in AmpliTaq Gold buffer (for concentrations see table 3. The following cycling conditions were used for all microsatellite primers: initial denaturation step 10 min at 95°C, followed by 35 cycles of 30 s 95°C, 30 s annealing at 56°C, 30 s at 72°C and a final extension step for 7 min at 72°C. Each PCR amplification was monitored by electrophoretic separation on a 2.5% agarose gel. Primer pairs were multiplexed. All PCR reactions were performed in independent triplets.

**Tab. 3: PCR-Samples of the Microsatellite-PCR-Screening ,**

| | | Samples: 1 | Samples: 10 |
|---|---|---|---|
| Reagents | Conc. | Vol. (µl) | Volume (µl) |
| PCR Gold Buffer | 10x | 1 | 10 |
| MgCl₂ | 25 mM | 0,8 | 8 |
| dNTP | 2,5 mM | 0,8 | 8 |
| Primer Forward | 10 µM | 0,1 | 1 |
| Primer Reverse | 10 µM | 0,1 | 1 |
| AmpliTaq Gold | 5 U/pl | 0,1 | 1 |
| DNA | 5 ng/µl | 4 | 40 |
| TMAC | 2mM | 0,5 | 5 |
| H₂O | ad. 30 µl | 2,6 | 26 |
| TOTAL | | 10 | 100 |

PCR products were diluted 5-fold in PCR-grade water (Merck), and 1 µl of each diluted sample was mixed with 0.15 µl of GeneScan-500-Rox size standard (Applied Biosystems) and 20 µl HIDI formamide. The mixtures were denatured at 95°C for 2 min and snap-cooled on ice. The fragments were separated by capillary electrophoresis ,(CE) and detected by laser induced fluorescence on an ABI PRISM 3100 Genetic Analyzer (Applied Biosystems). Two fragments were detected in the case heterocygocity, the informative case, differing in length from 2 to 10 base pairs. The Al status was determined by the ratio between allele 1 and 2. In the case of homocygocity only 1 fragment was measured and no information about the Al status of the cell could be obtained.

### Example 11

### REAL-TIME PCR

A real time PCR assay with SYBR green as a reporter was used to detect MDA-468 tumor cells via their increased gene doses for EGFR (epidermal growth factor receptor). For normalizing the gene doses, the housekeeping gene SOD2 (superoxid dismutase 2) was determined simultaneously. For the standard-concentration-curve for measuring the concentrations of the PCR products, leukocyte-DNA pre-amplified by WGSA was used as a template. All samples were measured in triplets..For amplification and data collection, the Mastercycler epgrandient S-realplex (Eppendorf) was used. All reactions were carried out in twintec PCR plates 96 (Eppendorf) in a total volume of 15 µl and sealed with optical adhesive covers (Applied Biosystems). For SYBR green assays the QuantiTect SYBR green Kit (Qiagen) was used with 1 µM of each of the primers provided in SEQ ID NO:3-6. The standard amplification protocol consists of an initial denaturation step at 95°C for 15 min, followed by 45 cycles of 95°C for 15 s, 58°C ,for 30 s and 68°C for 30 s. Fluorescence measurements were taken at the end of the elongation phases at 68°C. A melting curve step was added as the final step in each run to confirm specificity of PCR reaction. For each sample 10 ng DNA (2 µ3,5 ng/µl) were used as starting template. All samples and controls were measured in triplets. During the evaluation phase each amplification reaction of both assay types was checked for the absence of nonspecific PCR products by a native polyacrylamide gel electrophoresis.

Raw data were analyzed with the Realplex software Ver.2. For each primer pair a standard-concentration-curve was performed in each run using template amount of 10 ng, 2.5 ng, 0.625 ng and 0.156 ng. Leukocyte DNA or WGA product was used depending on the type of samples which were to be measured. The points of interception of the amplication-curves and the threshold (CT-value) were dependent on the amount of given template-DNA in their exponential regions. Using the assays with known amount of template-DNA calibration-lines (CT vs. Log2 of the amount of given template-DNA) were generated and their linear equations were calculated. To determine the amounts of DNA for the unknown samples the average for each triplicate of CT-values was calculated. The quotient of target against reference was then calculated giving the relative gene dosage of EGFR against LINE1. Standard deviations were calculated using the CV-method as described in Michael Walter Pfaffl (2004): Real-time RT-PCR: Neue Ansätze zur exakten mRNA Quantifizierung, Biospektrum BIOspektrum, 1/04, 10. Jahrgang, 92-95. Cancer cells were detected among peripheral blood cytokeratin-positive cells as cells displaying gene dosages for egfr greater than 2, thereby indicating gene amplification.

## Claims

1. Method for the enrichment and/or isolation of target cells - in a sample, which sample comprises red blood cells and/or platelets, comprising
(a) filtering the sample through a filter element having pores with a size of between 0.5 and 5 µm,
(b) contacting the cells retained by the filter element in step (a) with a separation surface, wherein said separation surface is a hydrogel coating on a support material and comprises affinity molecules which selectively bind to the target cells; ,
(c) incubating the cells and the separation surface under conditions which al.low for the binding of the affinity molecules to the target cells; and
(d) separating the separation surface from any unbound cells and material.

2. Method of claim 1, wherein the pore size of the filter element is between 1 and 2 µm.

3. Method of claim 1 or 2, wherein the filter element is a woven fabric.

4. Method of any of claims 1-3, wherein the target cells are tumor cells.

5. Method of any of claims 1-4, wherein the sample comprises whole blood, urine, pleural effusions, ascites, bronchoalveolar lavage, nipple aspirate of the gland of the female breast or bone marrow.

6. Method of any of the claims 1-5, wherein the support material is a slide, a bead or a chromatography resin.

7. Method of any of claims 1-6, wherein the affinity molecule is an antibody, an antigen-binding fragment of an antibody, a ligand, a lectin or a receptor or an aptamer.

8. Method of claim 7, wherein the affinity molecule is an antibody selected from the group consisting of an anti-EpCAM antibody, an anti-EGFR antibody, an anti-CD19 antibody, an anti-CK20 antibody, an anti-MUC1 antibody, an anti-MUC2 antibody, or an antigen-binding fragment thereof.

9. Method of any of claims 1-8, wherein the sample is diluted with a suitable buffer before or simultaneously with applying the sample to the filter element.

10. Method of any of claims 1-9, wherein the incubation in step (c) includes agitation of the separation surface.

11. Method of any of claims 1-10, wherein step (d) includes washing of the separation surface with a washing buffer.

12. Method for the detection and/or quantification of target cells in a sample, which sample comprises red blood cells and/or platelets, said method comprising
(a) performing a method according to any of claims 1-11 ; and
(b) detecting and/or quantifying the target cells.

13. Method of claim 12, wherein the detection and/or quantification is performed by use of detectably labeled antibodies or antibody fragments or by detectably labeled DNA probes.

14. Method of claim 12, wherein the detection and/or quantification involves a PCR, preferably a real-time PCR.

15. Method of claim 12, wherein the detection involves two-colour photoacoustic techniques.

## Patentansprüche

1. Verfahren zur Anreicherung und/oder Isolierung von Zielzellen in einer Probe, wobei die Probe rote Blutkörperchen und/oder Blutplättchen umfasst, Schritte umfassend, bei denen man:
(a) die Probe durch ein Filterelement filtriert, das Poren mit einer Größe zwischen 0,5 und 5 µm aufweist;
(b) die Zellen, welche durch das Filterelement in Schritt (a) zurückgehalten wurden, mit einer Separationsoberfläche in Kontakt bringt, wobei die Separationsoberfläche eine Hydrogel-Beschichtung auf einem Trägermaterial ist und Affinitätsmoleküle umfasst, die selektiv an die Zielzellen binden;
(c) die Zellen und die Separationsoberfläche unter Bedingungen inkubiert, die eine Bindung der Affinitätsmoleküle an die Zielzellen erlauben; und
(d) die Separationsoberfläche von ungebundenen Zellen und ungebundenem Material trennt.

2. Verfahren gemäß Anspruch 1, bei dem die Porengröße des Filterelements zwischen 1 und 2 pm liegt.

3. Verfahren gemäß Anspruch 1 oder 2, bei dem das Filterelement ein Gewebe ist.

4. Verfahren gemäß einem der Ansprüche 1-3, bei dem die Zielzellen Tumorzellen sind.

5. Verfahren gemäß einem der Ansprüche 1-4, bei dem die Probe Vollblut, Urin, Pleuraergüsse, Aszites, Bronchiallavage, Brustwarzenpunktat aus der Drüse der weiblichen Brust oder Knochenmark enthält.

6. Verfahren gemäß einem der Ansprüche 1-5, bei dem das Trägermaterial ein Objektträger, ein Bead oder ein Chromatographieharz ist.

7. Verfahren gemäß einem der Ansprüche 1-6, bei dem das Affinitätsmolekül ein Antikörper, ein Antigen-bindendes Fragment eines Antikörpers, ein Ligand, ein Lektin oder ein Rezeptor oder ein Aptamer ist.

8. Verfahren gemäß Anspruch 7, bei dem das Affinitätsmolekül ein Antikörper ist, der ausgewählt ist aus der Gruppe bestehend aus einem Anti-EpCAM-Antikörper, einem Anti-EGFR-Antikörper, einem Anti-CD19-Antikörper, einem Anti-CK20-Antikörper, einem Anti-MUC1-Antikörper, einem Anti-MUC2-Antikörper oder einem Antigen-bindenden Fragment derselben.

9. Verfahren gemäß einem der Ansprüche 1-8, bei dem die Probe vor oder gleichzeitig mit der Zuführung der Probe zum Filterelement mit einem geeigneten Puffer verdünnt wird.

10. Verfahren gemäß einem der Ansprüche 1-9, bei dem die Inkubation in Schritt (c) ein Bewegen der Separationsoberfläche umfasst.

11. Verfahren gemäß einem der Ansprüche 1-10, bei dem Schritt (d) das Waschen der Separationsoberfläche mit einem Waschpuffer umfasst.

12. Verfahren zum Nachweis und/oder zur Quantifizierung von Zielzellen in einer Probe, die rote Blutkörperchen und/oder Blutplättchen umfasst, Schritte umfassend, bei denen man:
(a) ein Verfahren gemäß einem der Ansprüche 1-11 durchführt; und
(b) die Zielzellen nachweist und/oder quantifiziert.

13. Verfahren gemäß Anspruch 12, bei dem der Nachweis und/oder die Quantifizierung unter Verwendung von nachweisbar markierten Antikörpern oder Antikörperfragmenten oder nach-weisbar markierten DNA-Sonden durchgeführt wird.

14. Verfahren gemäß Anspruch 12, bei dem der Nachweis und/oder die Quantifizierung eine PCR, vorzugsweise eine real-time-PCR umfasst.

15. Verfahren gemäß Anspruch 12, bei dem der Nachweis zweifarbige fotoakustische Techniken umfasst.

## Revendications

1. Procédé d'enrichissement d'un échantillon en cellules cibles et/ou d'isolement de cellules cibles dans un échantillon, lequel échantillon contient des globules rouges et/ou des plaquettes sanguines, et lequel procédé comporte les étapes suivantes :
a) filtrer l'échantillon en le faisant passer dans un élément filtrant doté de pores présentant une taille de 0,5 à 5 µm ;
b) mettre les cellules retenues par l'élément filtrant dans l'étape (a) en contact avec une surface pour séparation, laquelle surface pour séparation est un revêtement d'hydrogel disposé sur un matériau de support et comporte des molécules affinitaires qui se lient sélectivement aux cellules cibles ;
c) faire incuber les cellules cibles et la surface pour séparation dans des conditions qui permettent aux molécules affinitaires de se lier aux cellules cibles ;
d) et séparer la surface pour séparation d'avec toutes cellules non liées et d'avec tout matériau.

2. Procédé conforme à la revendication 1, dans lequel la taille des pores de l'élément filtrant vaut de 1 à 2 nm.

3. Procédé conforme à la revendication 1 ou 2, dans lequel l'élément filtrant est une étoffe tissée.

4. Procédé conforme à l'une des revendications 1 à 3, dans lequel les cellules cibles sont des cellules tumorales.

5. Procédé conforme à l'une des revendications 1 à 4, dans lequel l'échantillon comprend du sang complet, de l'urine, des épanchements pleuraux, un liquide d'ascite, un liquide de lavage broncho-alvéolaire, un liquide de glande mammaire aspiré par le mamelon, ou de la moelle osseuse.

6. Procédé conforme à l'une des revendications 1 à 5, dans lequel le matériau support est une lame, une perle ou une résine de chromatographie.

7. Procédé conforme à l'une des revendications 1 à 6, dans lequel la molécule affinitaire est un anticorps, un fragment d'anticorps se liant à l'antigène, un ligand, une lectine, un récepteur ou un aptamère.

8. Procédé conforme à la revendication 7, dans lequel la molécule affinitaire est un anticorps choisi dans l'ensemble formé par un anticorps anti-EpCAM, un anticorps anti-EGFR, un anticorps anti-CD19, un anticorps anti-CK20, un anticorps anti-MUC1 et un anticorps anti-MUC2, ou un fragment se liant à l'antigène d'un tel anticorps.

9. Procédé conforme à l'une des revendications 1 à 8, dans lequel, avant d'appliquer, ou en même temps qu'on applique, l'échantillon à l'élément filtrant, on dilue l'échantillon avec une solution tampon appropriée.

10. Procédé conforme à l'une des revendications 1 à 9, dans lequel l'opération d'incubation de l'étape (c) comporte le fait d'agiter la surface pour séparation.

11. Procédé conforme à l'une des revendications 1 à 10, dans lequel l'étape (d) comporte le fait de laver la surface pour séparation avec une solution tampon de lavage.

12. Procédé de détection et/ou de détermination de la quantité de cellules cibles dans un échantillon, lequel échantillon contient des globules rouges et/ou des plaquettes sanguines, et lequel procédé comporte les étapes suivantes :
a) mettre en oeuvre un procédé conforme à l'une des revendications 1 à 11 ;
b) et détecter les cellules cibles et/ou en déterminer la quantité.

13. Procédé conforme à la revendication 12, dans lequel on réalise la détection et/ou la détermination de quantité en utilisant des anticorps ou des fragments d'anticorps marqués de façon à être détectables, ou des sondes d'ADN marquées de façon à être détectables.

14. Procédé conforme à la revendication 12, dans lequel les opérations de détection et/ou de détermination de quantité impliquent une opération de PCR, et de préférence, de PCR en temps réel.

15. Procédé conforme à la revendication 12, dans lequel les opérations de détection impliquent des techniques photo-acoustiques en deux couleurs.
